# EUROPEAN PATENT APPLICATION

(11) **EP 1 239 397 A2**
(43) Date of publication of application: **11.09.2002**
(21) Application number: 02005135.5
(22) Date of filing: 07.03.2002
(51) Int. Cl.: G06F 19/00

(54) **Method of and system for reading medical image**

(30) Priority: 08.03.2001 JP 2001064553; 08.03.2001 JP 2001064554; 08.03.2001 JP 2001064555; 08.03.2001 JP 2001064556
(71) Applicant: Fuji Photo Film Co., Ltd., Kanagawa-ken (JP)
(72) Inventor: Kunimasa, Shimizu, Fuji Photo Co., Ltd., Tokyo 106-8620 (JP); Kazuhiro, Hishinuma, Fuji Photo Co., Ltd., Tokyo 106-8620 (JP)
(74) Representative: Klunker . Schmitt-Nilson . Hirsch

(57) **Abstract**

An image reading system includes clients which output image data as a visible image. Individual diagnoses obtained on the visible images are input through the client. A management client outputs result of examination obtained from the individual diagnoses. A server is provided with an image memory and a result memory connected to the clients and the management client by way of a network. The image memory stores image data and the result memory stores results of examination obtained on the individual diagnoses. Each client receives the image data from the server by way of a network, outputs the image data and sends individual diagnoses input for the image data to the server by way of the network. The result memory stores results of examination obtained on the individual diagnoses sent from the respective clients, and the management client outputs the result of examination received from the server.

## Description

### BACKGROUND OF THE INVENTION

### Field of the Invention

This invention relates to a method of and a system for reading a medical image.

### Description of the Related Art

In massive or personal medical checkup, a radiation image of a relevant part of a client is read by a doctor and whether reexamination is necessary is determined on the basis of the reading. In order to increase accuracy of the determination, it is often that a "multiple-reading method" is employed. In the multiple-reading method, two or more doctors separately read a radiation image and after the results of reading of all the doctors are output, a supervisor determines whether reexamination is necessary on the basis of the results of reading of all the doctors.

When reading medical images, the medical images are converted into medical image data by a digital image modality such as a CR and the medical image data is stored in a temporary memory such as a mass storage hard disc or a long-term memory such as a DLT and a DVD provided in a server. The medical image data stored in the server is transferred to a work station provided with a small storage temporary memory, a display and the like and the medical images are read by calling required pieces of image data from the small storage temporary memory and causing the display to reproduce the images represented by the pieces of image data. Generally, a chart and/or a report for each medical image is also stored in the small storage temporary memory of the work station and is called together with the corresponding piece of medical image data.

This system is advantageous when all the medical images to be read are read by one doctor. That is, when all the medical images to be read are read by one doctor, it is preferred that the medical images to be read be prevented from being viewed by other doctors until said one doctor finishes reading the images. Further, when one doctor reads all the medical images to be read with the medical images displayed as visible images in sequence, the above system is advantageous in view of efficiency.

However when the above system is employed in the multiple-reading method, the medical image data must be transferred to a plurality of work stations each provided with a small storage temporary memory, a display and the like. Further, when each doctor finishes reading the images, the doctor must inform the supervisor of the fact somehow, electronically, e.g., bye-mail, or non-electronically, e.g., verbally, and transfer to the supervisor a report or the like bearing thereon the result of his or her reading.

This system is disadvantageous in that the image data representing all the images to be read must be transferred to a plurality of temporary memories, which is wasteful of memory resources, and the supervisor must receive information on that each doctor finishes reading the images and the reports bearing thereon the result of his or her reading, which is troublesome for the supervisor.

Recently, there has been proposed a medical image network in which medical image data is concentrically stored in one memory (server) and is used by a plurality of clients. This medical image network comprises a server having a mass storage memory for storing medical image data and the like of patients and a plurality of clients connected to the server in a manner such that the medical image data stored in the server can be referred to through each client. With this medical image network, each client can display on its own display a desired piece of medical image data stored in the server. The server can store in a high-speed memory pieces of image data representing images which are more expected to be required by the clients and can archive image data representing images which are less expected to be required by the clients to a library which may comprise, for instance, magnetic tape or a DVD from which the image data is called at low speed.

For example, the image data generated is once stored in a hard disc, sent to a client as required from the client and is displayed for, for instance, diagnosis. Image data with which a certain time has elapsed from generation or a predetermined series of diagnosis has been carried out is sent to a library by a manual or automatic archive and stored in the library. With this arrangement, the image data can be concentrically stored in a server, and memory resources can be efficiently utilized. Further, since pieces of image data representing new images which are more expected to be required by the clients are stored in a hard disc, the time required for each client to call desired image data from the server can be shortened.

Further, there is a demand for the comparison of an old medical image and a new medical image in order to know course of a disease of a patient. In order to meet this demand, in the medical image network, it is possible to compress desired image data, e.g., pieces of image data with which a certain time has elapsed from generation or a predetermined series of diagnosis has been carried out, in different compression ratios and a couple of pieces of image data obtained by compressing the same image data in different compression ratios may be respectively stored in a library and a hard disc. For example, a piece of image data obtained by compressing a desired image data in a lower compression ratio may be semi-permanently stored in the library with a piece of image data obtained by compressing the desired image data in a higher compression ratio (the compression ratio being selected to conform to the purpose) temporarily stored in the hard disc. That is, when the image data is only to be attached to the chart, for instance, image data compressed in an irreversible compression system in a relatively higher compression ratio may be stored in the hard disc, while when the image data is to be used in observing the course of a disease, image data compressed in an irreversible compression system in a relatively lower compression ratio may be stored in the hard disc. With this arrangement, image data of a desired property can be constantly efficiently called through each client. That is, image data of a high necessity can be called at a high speed, and image data of a relatively low necessity can be called at a relatively low speed.

As a system utilizing the medical image network described above, there has been known a medical architecture system or an external observation method in which medical images and/or data on a patient can be called from a site remote from the server and observation of a different doctor at a distant place can be quickly obtained. (See, for instance, Japanese Unexamined Patent Publication No. 11(1999)-161729.)

### SUMMARY OF THE INVENTION

In view of the foregoing observations and description, a first object of the present invention is to provide a medical image reading system and a medical image reading method which make it feasible to carry out the multiple-reading method utilizing the medical image network described above so that the memory resources can be efficiently utilized and the supervisor can efficiently make final determination.

Medical images such as X-rays, CTs (computerized axial tomograms), MRIs (magnetic resonance images) and the like should be stored in order to know course of diseases of patients and medical facilities such as physicians, hospitals and the like are legally obliged to store medical images for a predetermined time. Accordingly, in the medical facilities, the number of medical images to be stored increases day by day. Conventionally, medical images have been stored in the form of hard copies, keeping spaces for medical images, management of the same and reference of the same have been heavy load on the medical facilities.

There has been known a so-called image file in which digital image data is generated from a digital image or the like and filed to be retrievable in a recording medium such as a magnetic disc. When medical images are stored in the recording medium by the use of such an image file, the space required to store images can be saved, the manpower required to store images can be saved and at the same time, retrieval of images is facilitated and can be done at a high speed.

However, the digital image data generating apparatus and the image file are very expensive and occupies a large space. Accordingly, it is difficult for a relatively small medical facility such as a physician's office to be equipped with the digital image data generating apparatus and the image file.

In order to overcome this problem, there has been proposed a service in which hard copies of medical images are non-electronically transferred to a data center from medical facilities, digital image data representing the medical images are obtained at the data center by the use of, for instance, a film digitizer and the digital image data is stored in a mass storage memory equipped in the data center. The data center outputs a required image on demand from a medical facility as a hard copy recorded on a film. When the medical facility is equipped with a work station which can be connected to the mass storage memory in the data center through a network, the medical facility can directly access to the mass storage memory through the work station to download the image data representing the desired image. The medical facilities are charged for using a storage space in the mass storage memory and use of image output service on the basis of the storage capacity or the number of times of outputs.

When this service is used, the space for storing medical images ;can be saved without necessity of purchasing expensive equipments, the manpower required to store and manage the medical images can be saved, and retrieval of images can be done at a high speed.

In the service described above, though medical images are digitized and stored in the data center, when a medical facility is not equipped with a means for calling image data, the medical facility must make a diagnosis on the basis of a hard copy of the medical image output on a film, which means that the medical facility cannot enjoy advantages inherent to the digital image system, for instance, that the medical image can be processed according to the content of diagnosis or a desired medical image can be easily searched for among a lot of medical images . Even if a medical facility is equipped with a means for calling image data, the medical facility cannot enjoy advantages inherent to the digital image system until the medical image is digitized and stored in the data center. Since it takes a certain time to digitize an image and store the digitized image data in the data center, the medical facility cannot enjoy advantages inherent to the digital image system as for images which are taken on that day and generally read before they are delivered to the data center. However, since medical images taken on that day are read in detail in order to make a diagnosis, it is preferred that image processing for improving diagnostic performance of the medical images be carried out on the medical images.

Further, the service is disadvantageous in that since medical images are non-electronically transferred to a data center from medical facilities, the data center must have charge of receiving the medical images and digitizing them and must have charge of manually executing accounting processing.

A second object of the present invention is to provide a centralized medical image storing method of and a centralized medical image storing system which make it feasible for medical facilities to enjoy advantages inherent to the digital image system for all the medical images to be used in diagnosis, can reduce load on the data center and make it feasible for the data center to automatically executing accounting processing.

Further, in the service described above, the medical facilities are charged on use of image output service on the basis of the number of images output. However this accounting system is unreasonable in that the number of images output largely differs according to the kind of medical examination and/or the part to be examined. For example, in a medical examination of the chest, one or two images is necessary, in a medical examination of the urinary organs, about five images are necessary, and in a medical examination of the digestive organs, about ten images are necessary. Thus, the charge for one examination largely differs according to the kind of examination.

A third object of the present invention is to provide a medical information storing and accounting system and a method of accounting for output of medical information from a server which can reasonably account for output of medical information from the server.

Recently, an increasing number of medical facilities have come to use an electronic patient's chart representing clinical information on the patient. Clinical information obtained by reading a medical image of the patient is often recorded in the electronic patient's chart for the patient. Since the contents of the electronic patient's charts are high secrets and the electronic patient's chart can be stored in a memory which need not be so large in capacity, even medical facilities which store medical images in the data center generally store electronic patient's charts in their own memories.

Since the mass storage memory in the data center in which medical images are stored is not connected with the memories in the medical facilities in which the electronic patient's charts are stored, it has been difficult to relate the electronic patient's chart with the medical images. That is, when making a diagnosis on the basis of medical images, an electronic patient's chart is first displayed on a display of a work station in the medical facility and medical images to be read are determined on the basis of the electronic patient's chart. Then the data center is requested to electronically or non-electronically output the images. A doctor in the medical facility checks that the images output from the data center correctly correspond to the requested images, reads the images and makes a diagnosis on the basis of reading of the images. Then the doctor records the result of diagnosis in the corresponding patient's chart through the work station. Accordingly, even in a medical facility equipped with a work station which can download medical image data from the data center, electronic patient's chart and medical images related to each other must be manually called from the respective memories each time a diagnosis based on medical images is made, which is very inefficient.

A fourth object of the present invention is to provide a medical information output system and method which can improve image reading efficiency when electronic patient's chart are stored in each medical facilities and medical images are stored in a data center.

The first object of the present invention can be accomplished by a medical image reading system comprising
a plurality of diagnostic clients provided with an image output means which outputs image data to be examined as a visible image, and a diagnosis input means for inputting individual diagnoses obtained on the basis of visible images,
at least one management client provided with a result output means for outputting result of examination obtained on the basis of the individual diagnoses, and
a server provided with an image storage means and a result storage means which are connected to the diagnostic clients and the management client by way of a network, the image storage means storing image data to be examined and the result storage means storing results of examination obtained on the basis of the individual diagnoses with the results of examination related to the image data, wherein
each of the diagnostic clients receives the image data to be examined from the server by way of a network, outputs the image data to be examined through the image output means and sends individual diagnoses input through the diagnosis input means for the respective images represented by the image data to be examined to the server by way of the network,
the server causes the result storage means to store results of examination obtained on the basis of the individual diagnoses sent from the respective diagnostic clients, and
the management client receives the result of examination from the server by way of the network and causes the result output means to output the same.

The term "image data to be examined" as used here means image data representing an image or images to be examined. That is, the "image data to be examined" may comprise a piece of image data representing an image or a plurality of pieces of image data representing a plurality of images of one object which are taken at one time or different times.

Further, the "image output means" provided in the diagnostic client or the "result output means" provided in the management client is generally a display means such as a CRT but may be a hard copy output means such as a printer.

The expression "storing results of examination obtained on the basis of the individual diagnoses with the results of examination related to the image data" means to store results of examination obtained on the basis of the individual diagnoses input from the diagnostic clients for a certain pieces of image data so that the results of examination and the pieces of image data can be related to each other in one-to-one correspondence. For example, the former and the latter may be stored in a memory together each other or may be stored in different memories logically related to each other. That is, the image storage means and the result storage means may be the same or different.

The term "results of examination obtained on the basis of the individual diagnoses" may be the individual diagnoses sent from the diagnostic clients as themselves or result of examination automatically obtained on the basis of the individual diagnoses. That is, the server may further comprise an automatic determination means which automatically makes examination for a given piece of image data on the basis of all or part of the individual diagnoses received and outputs result of examination.

Further, each of the diagnostic clients may be arranged to be able to send data on the doctor in charge together with the relevant individual diagnosis to the server so that the automatic determination means makes examination weighting the individual diagnoses according to the doctor in charge. The result of examination may be in the form of any data so long as it can represent the result of examination. For example the result of examination may represent the respective individual diagnoses, values representing the degree of necessity of reexamination, their various combinations or result of decision by majority.

The server may be further provided with an informing means which, when the server receives a predetermined number of said diagnoses, sends information to the effect that the server has received a predetermined number of said diagnoses to the management client and the management client may be provided with an information output means which receives the information and outputs the same.

The "predetermined number of said diagnoses" may be either of all or part of the individual diagnoses for a given piece of image data but may be, for instance, a plurality of individual diagnoses for different pieces of image data. The information output means is generally a display means such as a CRT but may be a hard copy output means such as a printer.

The network as used in the medical image reading system is preferably the aforesaid medical image network (e.g., a LAN such as Ethernet) but may be other various networks such as an internet, a private line and an inter-hospital network.

The management client need not be separate from the diagnostic clients. For example, the management client may be incorporated in one or more of diagnostic clients by adding the function of the management client to one or more of the diagnostic clients.

The first object of the present invention can also be accomplished by a medical image reading method comprising the steps of
storing image data to be examined in a server which is provided at a place remote from a plurality of diagnostic clients and connected to the diagnostic clients by way of a network,
causing each of the diagnostic clients to receive a desired piece of image data out of the image data stored in the server and to output the desired piece of image data as a visible image,
inputting individual diagnoses obtained on the basis of the output visible image through the diagnostic clients,
sending the individual diagnoses to the server,
storing in the server results of examination obtained on the basis of the individual diagnoses sent thereto with the results of examination related to the pieces of image data, and
causing a management client to receive the result of examination for a desired piece of image data stored in the server and to output the same.

With the medical image reading system and method for accomplishing the first object of the present invention, since each of the diagnostic clients receives the image data to be examined from the server by way of a network and outputs the image data to be examined, it is not necessary to provide each diagnostic client with a temporary memory and the memory resources can be efficiently utilized.

Further, since individual diagnoses from the plurality of diagnostic clients are all stored in the server, the management client can receive result of examination obtained on the basis of the individual diagnoses, the supervisor need not receive information on that each doctor finishes reading the images and the reports bearing thereon the result of his or her reading, whereby final determination can be made efficiently.

When the server is arranged, when the server receives all of said diagnoses, to send information to the effect that the server has received all of said diagnoses to the management client, efficiency of examination is further enhanced.

Further when the server further comprises an automatic determination means which automatically makes examination for a given piece of image data on the basis of all or part of the individual diagnoses received and outputs result of examination, efficiency of examination is further more enhanced.

The second object of the present invention can be accomplished by a centralized medical image storing system comprising
a plurality of clients each of which is provided in a medical facility, equipped with an image input means for inputting medical image data obtained by a digital image data generating apparatus, and transfers medical image data by way of a network, and
a server which is connected to the clients by way of the network and provided with an image storage means and an online accounting means, the image storage means storing medical image data sent from the clients and the online accounting means automatically outputting accounting information for medical facilities,
wherein the improvement comprises that
each of the clients is provided with a function of sending a request for receiving a desired piece of medical image data out of image data stored in the image storage means to the server, the server is provided with a function of sending the desired piece of image data to the client which sends the request in response to receipt of the request, and each of the clients is provided with an image output means which outputs as a visible image the desired image data sent from the server.

The image output means may output as a visible image the medical image data input through the image input means.

The term "accounting information" as used here means various charges on the medical facility and includes, for instance, storage cost for the medical image data which the medical facility holds in possession, a rent for a digital image generating apparatus and/or a client which is rented to the medical facility and a data fee imposed on the medical image data used through the client. That is, the accounting information is information on the sum of the charges on the medical facility.

The storage cost includes, for instance, a charge for using the storage space, a charge for maintenance of medical image data stored in the image storage means, and a service charge for deleting medical image data. The "charge for using the storage space" may be calculated, for instance, by summing the image sizes represented by pieces of image data stored in the image storage means or may be an annual fee for a predetermined storage space. The "charge for maintenance of medical image data" is cost for managing the image data such as for changing the storage medium of the image data and may be a fixed value image by image. Otherwise the charge for maintenance of medical image data may be determined according to the period of time for which the image data has been stored. For example, the charge for maintenance of medical image data may be determined for a period not longer than three years, a period of three to five years, a period of five to ten years, and so on. The charge for maintenance of medical image data may be increased with increase in the period of time for which the image data has been stored. The "service charge for deleting medical image data" may be cost of deleting the image data and/or reporting the fact and may be determined image by image.

The "rent for a digital image generating apparatus and/or a client" is charged for the part of the digital image generating apparatus and/or the client which is rented to the medical facility and for other components rented to the medical facility. The means for connecting the client to the network (e.g., a modem) is a part of the client irrespective of whether it is built-in or not.

The "data fee" is imposed on the medical image data which is stored in the storage means and used through the client. The data fee may be charged in various systems, e.g., may be charged according to the number of images used, or the size of the image used.

The "online accounting means" automatically calculates accounting information for medical facilities.

The "network "as used here may be various networks such as an internet, a private line and the like.

The "request for receiving a desired piece of medical image data" includes information on the medical image data which is to be sent from the server. The "desired piece of image data" need not be those transferred to the server from the client but may be those transferred to the server from other medical facilities.

Further, the "image output means" is preferably a display means such as a monitor but may be any means such as a printer so long as it can output the medical image data as a visible image.

The storage means may comprise a high-speed storage means and a low-speed storage mean. The high-speed storage means may be a hard disc, a RAID disc or the like which can transfer the medical image data stored therein to the client at a high speed. The low-speed storage means may be, for instance, a mass storage memory such as a library using DLD or DVD which can transfer the medical image data stored therein to the client at a low speed.

The client used in the centralized medical image storing system for accomplishing the second object of the present invention comprises an image input means for inputting medical image data obtained by a digital image data generating apparatus, a first data transfer means which transfers the medical image data input through the image input means to a sever by way of a network, a second data transfer means which sends a request for receiving a desired piece of medical image data out of image data stored in the server to the server, a data receiving means which receives from the sever the desired piece of medical image data as based on the request sent from the second data transfer means, and an image output means which outputs as a visible image the desired piece of medical image data received by the data receiving means.

The server used in the centralized medical image storing system for accomplishing the second object of the present invention comprises a first data receiving means which receives medical image data from a plurality of clients by way of a network, a storage means which stores the medical image data received by the first data receiving means, a second data receiving means which receives a request for receiving a desired piece of medical image data out of image data stored in the server from the clients, a data transfer means which transfers to the clients the desired piece of medical image data as based on the request received by the second data receiving means, and an online accounting means which automatically outputs accounting information for medical facilities equipped with the clients.

The second object of the present invention can also be accomplished by a centralized medical image storing method comprising the steps of
inputting medical image data obtained by a digital image data generating apparatus through a plurality of clients each of which is provided in a medical facility, transferring the medical image data to a server by way of a network, storing the medical image data in the server, causing the clients to send a request for receiving a desired piece of medical image data out of image data stored in the server to the server, causing the server to send the desired piece of image data to the client which sends the request in response to receipt of the request, causing the clients to output as a visible image the desired image data sent from the server, and causing the server to automatically output accounting information for medical facilities.

With the centralized medical image storing system and method!for accomplishing the second object of the present invention, up-to-date images can be obtained at each of the medical facilities not as a hard copy but as image data. Accordingly, the image can be subjected to, for instance, image processing for improving the diagnostic performance. Further, each medical facility can use all the image data stored in the server and can enjoy advantages inherent to the digital image system for all the medical images.

When the digital image data generating apparatus and/or the client are rented for a counter value, the medical facility need not purchase expensive equipment. That is, it has been difficult for small scale medical facilities to be equipped with the digital image data generating apparatus and the like which are expensive. However, by renting such equipments, even a small scale medical facility can enjoy advantages inherent to the digital image system.

Further, since the clients provided in the medical facilities are connected to the server by way of a network, it becomes feasible to send medical image data to the sever from the clients to directly store the image data in the server, whereby load on the server side e.g., charge of receiving the medical images, can be lightened and the labor cost can be reduced.

Further since the image data can be electronically sent and received and accounting processing can be automatically executed, which makes online accounting feasible.

When the medical image data input into the client from the digital image generating means is output as a visible image before sent to the server, it becomes feasible to check the image before sending the medical image data to the server, it becomes feasible to view a medical image to be viewed instantly and image data which became unnecessary due to errors in taking images can be omitted before actually sending image data to the server, whereby the time required to send image data to the server and/or the capacity of image data to be stored in the server, which governs the storage space fee, the service charge for deleting medical image data and the like, can be saved.

When the storage means provided in the server comprises a high-speed storage means and a low-speed storage mean, a larger storage space can be ensured. Further when image data representing images which are more expected to be required by the clients is stored in the high-speed storage means and image data representing images which are less expected to be required by the clients is stored in the low-speed storage means, transfer of image data on demand from the clients can be done more efficiently.

The third object of the present invention can be accomplished by a medical information storing and accounting system comprising
a plurality of clients each of which is provided in a medical facility, receives medical image data by way of a network and is provided with an image output means which outputs as a visible image the medical image data received, and
a server which is connected to the clients by way of the network and provided with an image storage means which stores medical image data and an online accounting means which automatically outputs accounting information for medical facilities,
wherein the improvement comprises that
each of the clients is provided with a function of sending a request for receiving medical image data related to a predetermined examination out of image data stored in the image storage means to the server,
the server is provided with a function of sending, to the client which sends the request, image data as requested by the client, and
the accounting information output by the online accounting means includes a data fee determined examination by examination.

That is, the medical information storing and accounting system for accomplishing the third object of the present invention is characterized in that the data fee included in the accounting information output by the online accounting means is determined examination by examination.

The term "accounting information" as used here means various charges on the medical facility and includes, for instance, storage cost for the medical image data which the medical facility holds in possession and a data fee imposed on the medical image data used through the client. That is, the accounting information is information on the sum of the charges on the medical facility.

The "request for receiving medical image data related to a predetermined examination" as used here means a signal which requests the server to send one or more piece of image data representing one or more images related to a relevant examination (e.g., latest images of an object and past images of the same object) and includes information to identify the relevant examination or medical image data related to the relevant examination. This request will be referred to as "medial image receiving request", hereinbelow.

The "online accounting means" automatically calculates accounting information for medical facilities.

The "image output means" is preferably a display means such as a monitor but may be any means such as a printer so long as it can output the medical image data as a visible image.

The "network "as used here may be various networks such as an internet, a private line and the like.

In the medical information storing and accounting system for accomplishing the third object of the present invention, each of the clients may be arranged to send, to the server, purpose information representing the purpose of examination together with the request for receiving medical image data related to the predetermined examination and the server may be arranged to send to the client medical image data related to the predetermined examination within an available range determined purpose by purpose on the basis of the purpose information with the data fee determined by purpose of examination.

The expression "to send to the client medical image data related to the predetermined examination within an available range determined purpose by purpose" as used here means to send image data within an available range determined according to the purpose of examination when the server sends medical image
data in response to the medical image receiving request. For example, when the medical image receiving request sent from a client includes information to identify medical image data to be sent (e.g. an image data reference number), the server may be arranged to send only the medical image data identified by the medical image receiving request even if there are other image data related to the examination. Otherwise, when the medical image receiving request sent from a client does not include information to identify medical image data to be sent (e.g. an image data reference number) but includes information to identify an examination (e.g. an examination reference number), the server may be arranged to send medical image data related to the examination within the available range according to the purpose of examination. For example, when the purpose of examination is diagnosis, the server may send latest images of the object and past images of the same and when the purpose of examination is reference, the server may send one of the latest images of the object and the past images of the same.

The "available range" may be a range related to one or more of the kind of images, the pixel density of images and image processing right. The "kind of images" represents latest images or past images, or which of images of the same object, the pixel density represents whether the images are high or low in pixel density, and the image processing right represents the right to carry out image processing. The aforesaid "available range" may be, for instance, a range which is determined purpose by purpose on the basis of only the kind of images (e.g., only latest images are available or all the images are available, or on the basis of the pixel density (e.g., only images which are low in pixel density are available or only images which are in a predetermined range in pixel density are available).

In the medical information storing and accounting system for accomplishing the third object of the present invention, the server may further comprise a report storage means which stores a report on the medical image data, each of the clients may further comprise a report output means which receives a report stored in the report storage means and outputs it and the server may send, to the client, image data as requested by the client together with the report on the medical image data.

The report on image data may be, for instance, diagnosis made on the basis of the image and may be in the form of an electronic patient's chart.

Further, the server may further comprise a report storage means which stores a report on the medical image data, each of the clients may further comprise a report output means which receives a report stored in the report storage means and outputs it, a report input means for inputting a report and a function of sending, to the sever, the report input through the report input means, the server may send, to the client, image data as requested by the client together with the report on the medical image data, and the available range may be a range related to one or more of the kind of images, the pixel density of images, image processing right and report input right.

The client used in the medical information storing and accounting system for accomplishing the third object of the present invention comprises a data sending means which sends a request for receiving medical image data related to a predetermined examination out of image data stored in a server connected thereto by way of a network, a data receiving means which receives, from the server, medical image data as requested by the request for receiving medical image data sent by the data sending means, and an image output means which outputs as a visible image the medical image data received by the data receiving means.

The server used in the medical information storing and accounting system for accomplishing the third object of the present invention comprises
an image storage means which stores medical image data,
a data receiving means which receives a request for receiving medical image data related to a predetermined examination out of image data stored in the image storage means from a client connected thereto by way of a network,
a data sending means which sends, to the client, medical image data as requested by the request for receiving medical image data received by the data receiving means, and
an online accounting means which automatically outputs accounting information for the medical facility equipped with the client, the accounting information including a data fee determined examination by examination.

The third object of the present invention can also be accomplished by a medical information storing and accounting method comprising the steps of
storing image data in a server connected by way of a network to a plurality of clients each of which is provided in a medical facility,
sending, from the server to the client which sends a request for receiving medical image data related to a predetermined examination out of image data stored in the server, image data as requested by the client,
outputting through the client the medical image data sent by the server, and
causing the server to automatically output accounting information for medical facilities, the accounting information including a data fee determined examination by examination.

It is possible to determine an available range purpose by purpose and to determine the data fee examination by examination and purpose by purpose.

It is further possible to store image reports on the medical image data in the server and send, to the client, image data as requested by the client together with the report on the medical image data.

With the medical information storing and accounting system and method for accomplishing the third object of the present invention, since the data fee on the medical facilities is determined examination by examination, the charge for one examination does not largely differ even if the number of images to be referred to fluctuates according to the kind of examination, which is reasonable.

Further, when an available range is determined purpose by purpose and the data fee is determined examination by examination and purpose by purpose, it becomes feasible to determine the available range and/or the charge according to the purpose, which is further reasonable. That is, in the conventional system, for example, though necessary image quality differs purpose by purpose, the quality of image sent to the medical facility is constant irrespective of the purpose and the charge is fixed, which is unreasonable. For example, when an image is used for the purpose of diagnosis, a high-density image is necessary. In such a case, the available range is set so that a high-density image can be used and the account is set high. To the contrast, for example, when an image is used only to refer to the examination history of the patient checking his or her chart and past images, the image may be of relatively low quality. In such a case, the available range is set so that only a low-density image can be used and the account is set low. Thus, the medical information storing and accounting system and method for accomplishing the third object of the present invention can reasonably account for output of medical information from the server.

The fourth object of the present invention can be accomplished by a medical information output system comprising
a plurality of clients each of which is equipped in a medical facility and is provided with an image output means which receives medical image data by way of a network and outputs as a visible image the medical image data received, an electronic patient's chart storage means which stores data on the patient's chart, and an electronic patient's chart output means which outputs data on the patient's chart stored in the electronic patient's chart storage means, and
a server which is connected to the clients by way of the network and is provided with an image storage means which stores medical image data, wherein the improvement comprises that
each of the clients is provided with a liaison output means which causes the electronic patient's chart output means and the image output means to output in liaison with each other, and the liaison output means causes the electronic patient's chart output means and the image output means to output data on the patient's chart and medical image data corresponding to the patient's chart by the use of liaison information which relates the patient's chart and the medical image data with each other.

The "liaison output means" as used here may be any means so long as it can which cause the electronic patient's chart output means and the image output means to output data on the patient's chart and medical image data corresponding to the patient's chart in liaison with each other. For example, those using a web link which has been used on the internet web browser may be used. That is, the clients and the server are connected by an internet so that, when each client receives medical image data stored in the server, the client receives the medical image data using the internet web browser and electronic patient's chart software which operates on the web browser is installed in the clients so that the electronic patient's chart is output onto the Web browser, the address on the Web of the medical image data corresponding to the electronic patient's chart output onto the Web browser is found, for instance, by the use of liaison information which relates the patient's chart and the medical image data with each other and the browser screen is opened. In this case, a system which causes the electronic patient's chart output means and the image output means to output data on the patient's chart and medical image data corresponding to the patient's chart may be used as the liaison output means.

The "liaison information which relates the patient's chart and the medical image data with each other" may be information included in the patient's chart or the medical image data or may be information stored in the client or the server separately from the patient's chart and the medical image data. For example, the liaison information may be a reference number of a medical image included in the patient's chart, the same reference code included in both the patient's chart and the medical image data corresponding to the patient's chart, or information relating the number of patient's chart included in the chart to the number of medical image corresponding to the chart. When the liaison information includes information stored in the server, the client receives the information from the server by way of the network and uses it. For example, when the client receives a desired piece of image data from the server, the server may send the liaison information to the client together with the medical image data.

The medical information output system for accomplishing the fourth object of the present invention may be arranged so that
each of the clients is provided with a function of sending a request for receiving medical image data out of image data stored in the image storage means to the server,
the server is provided with a function of sending image data to the client which sends the request in response to receipt of the request, and
the liaison output means selects medical image data on the basis of the patient's chart output from the electronic patient's chart output means by the use of liaison information, sends a request for receiving selected medical image data to the server and causes the image output means to output the medical image data sent from the server upon receipt of the request.

Further, the medical information output system for accomplishing the fourth object of the present invention may be arranged so that
each of the clients is provided with a function of sending a request for receiving medical image data out of image data stored in the image storage means to the server,
the server is provided with a function of sending image data to the client which sends the request in response to receipt of the request, and
the liaison output means selects patient's chart on the basis of the medical image data received from the server by the use of liaison information, and causes the electronic patient's chart output means to output the selected patient's chart.

Further, the medical information output system for accomplishing the fourth object of the present invention may be arranged so that
each of the clients is provided with a function of sending a request for receiving medical image data out of image data stored in the image storage means to the server,
the server is provided with a function of sending image data to the client which sends the request in response to receipt of the request, and
the liaison output means is provided with a function of selecting medical image data on the basis of the patient' s chart output from the electronic patient's chart output means by the use of liaison information, sending a request for receiving selected medical image data to the server and causing the image output means to output the medical image data sent from the server upon receipt of the request and a function of selecting patient's chart on the basis of the medical image data received from the server by the use of liaison information and causing the electronic patient's chart output means to output the selected patient's chart.

The "request for receiving selected medical image data" includes information to identify medical image data.

The client used in the medical information output system for accomplishing the fourth object of the present invention comprises
an electronic patient's chart storage means which stores data on the patient's chart, an electronic patient's chart output means which outputs data on the patient's chart stored in the electronic patient's chart storage means, a data sending means which sends a request for receiving medical image data out of image data stored in a server connected thereto by way of a network, a data receiving means which receives, from the server, medical image data as requested by the request for receiving medical image data sent by the data sending means, an image output means which outputs as a visible image the medical image data received by the data receiving means, and a liaison output means which causes the electronic patient's chart output means and the image output means to output in liaison with each other, wherein
the liaison output means is provided with at least one of a function of selecting medical image data on the basis of the patient's chart output from the electronic patient's chart output means by the use of liaison information, sending a request for receiving selected medical image data to the server and causing the image output means to output the medical image data sent from the server upon receipt of the request and a function of selecting patient's chart on the basis of the medical image data received from the server by the use of liaison information and causing the electronic patient's chart output means to output the selected patient's chart.

The server used in the medical information output system for accomplishing the fourth object of the present invention comprises
an image storage means which stores medical image data,
a data receiving means which receives a request for receiving medical image data related to a predetermined examination out of image data stored in the image storage means from a client connected thereto by way of a network, and
a data sending means which sends, to the server, medical image data as requested by the request for receiving medical image data received by the data receiving means.

The fourth object of the present invention can also be accomplished by a medical information output method comprising the steps of
storing data on patient's charts in each of clients equipped in medical facilities,
storing medical image data related to the patient's charts in a server connected to the clients by way of a network, and
causing each of the clients to automatically receive medical image data related to data on a patient's chart to be output and to output the medical image data together with the patient's chart.

The fourth object of the present invention can also be accomplished by a medical information output method comprising the steps of
storing data on patient's charts in each of clients equipped in medical facilities,
storing medical image data related to the patient's charts in a server connected to the clients by way of a network, and
causing each of the clients to automatically select data on a patient's chart related to medical image data to be output and to output the selected patient's chart together with the medical image data.

In the medical information output system and method for accomplishing the fourth object of the present invention, since, in a system where electronic patient's charts are stored
in a client equipped in a medical facility and medical image data is stored in a server, a liaison output means which causes an electronic patient's chart output means and an image output means to output an electronic patient's chart and medical image data in liaison with each other by the use of liaison information is provided, diagnosis can be efficiently made without necessity of manually selecting medical image data and electronic patient's chart related with each other.

When each of the clients is provided with both a function of receiving medical image data, from the server, related to an output electronic patient's chart and outputting the medical image data and a function of outputting an electronic patient's chart related to medical image data received from the server, the patient's chart and the medical image data can be alternately output according to, for instance, the purpose of diagnosis or request and accordingly efficiency of diagnosis can be further enhanced. That is, the medical image data and the patient's charts can be displayed in sequence suitable for the purpose, e.g., when reading a medical image designated by an electronic patient's chart or when recording result of diagnosis in corresponding patient's charts while reading images of a plurality of objects in massive medical checkup, which leads to enhancement of the diagnostic efficiency.

### BRIEF DESCRIPTION OF THE DRAWINGS

Figure 1 is a schematic view showing a medical image reading system in accordance with a first embodiment of the present invention,
Figure 2 is a schematic view for illustrating the arrangement of the server,
Figure 3 is a flow chart for illustrating the operation of the medical image reading system,
Figure 4 is a schematic view showing a second embodiment of the present invention which embodies a centralized medical image storing system or a medical information output system in accordance with the present invention,
Figure 5 is a schematic view for illustrating the arrangement of the client and the server employed in the second embodiment,
Figure 6 is a flow chart for illustrating the processing of using image data in the second embodiment,
Figure 7 is a schematic view showing a medical information storing and accounting system in accordance with a third embodiment of the present invention,
Figure 8 is a schematic view for illustrating the arrangement of the client and the server employed in the third embodiment, and
Figure 9 is a flow chart for illustrating the processing of using image data in the third embodiment.

### DESCRIPTION OF THE PREFERRED EMBODIMENT

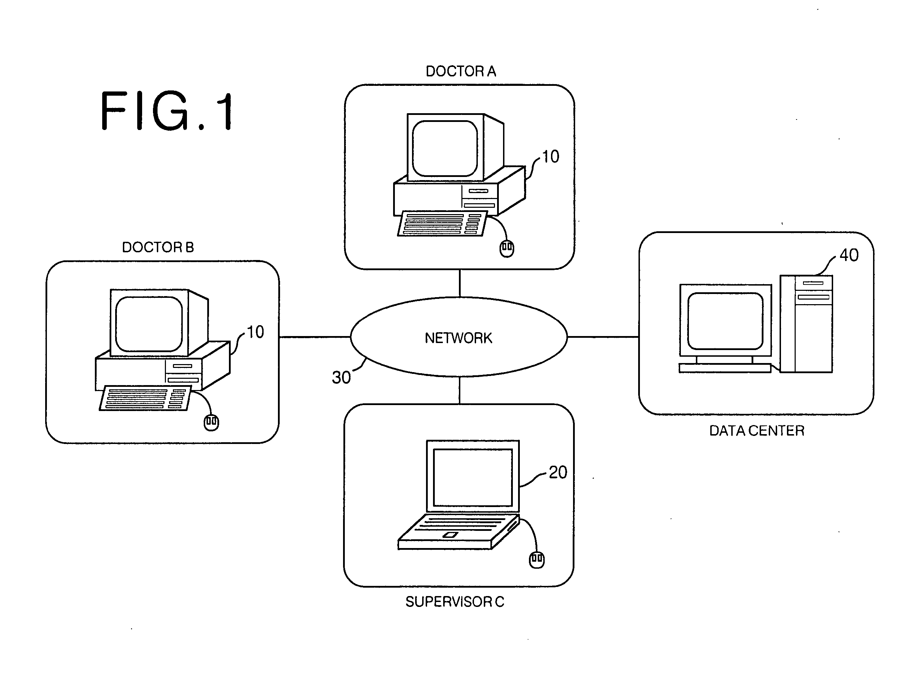

In Figure 1, a medical image reading system in accordance with a first embodiment of the present invention comprises a
plurality of diagnostic clients 10 provided by a plurality of doctors, a management client 20 provided by a supervisor and a server 40 which is equipped in a data center and connected to the diagnostic clients 10 and the management client 20 by way of;a network 30.

Each of the diagnostic clients 10 is provided with an input system comprising, for instance, a mouse and a keyboard, and a monitor. The monitor is an image output means which displays as a visible image the image data to be examined, and the input system is an input means through which result of diagnosis made by the doctor on the basis of the image displayed on the monitor.

The management client 20 is provided, similarly to the diagnostic client 10, with an input system comprising, for instance, a mouse and a keyboard, and a monitor. The input system is used, for instance, for calling result of examination from the server 40 and the monitor is used as a result output means for outputting the result of examination. Though, in Figure 1, the diagnostic clients 10 and the management client 20 are respectively in the form of a desktop computer and a notebook size personal computer, they need not necessarily be so.

The diagnostic clients 10 and the management client 20 are respectively connected to the network 30 and are respectively provided with a data sending means which sends data to the server 40 by way of the network 30 and a data receiving means which receives data from the server 40 by way of the network 30.

The network 30 connects the clients and the server 40 so that various pieces of data such as medical image data, diagnostic data and data representing information can be transferred between the clients and the server 40.

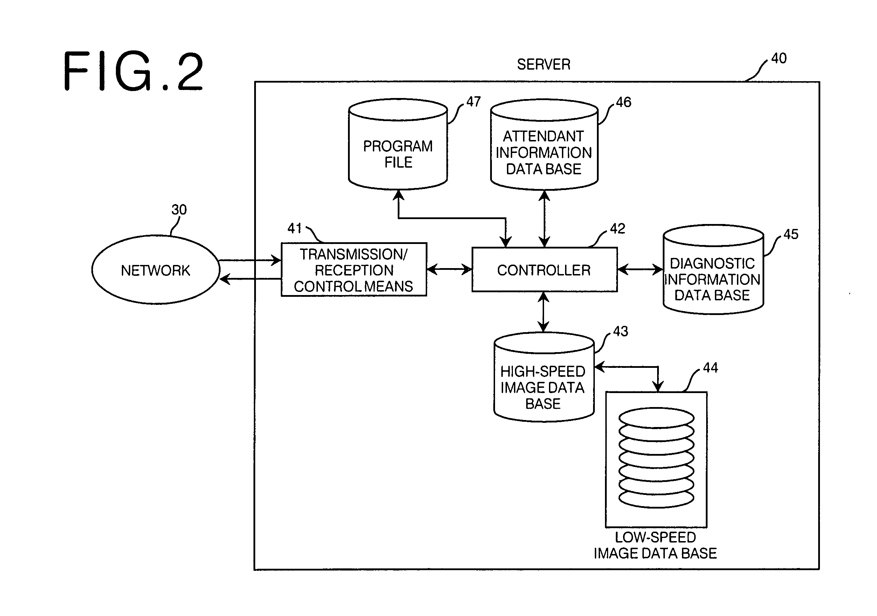

As shown in Figure 2, the server 40 comprises a transmission/reception control means 41, a controller 42, a high-speed image data base 43, a low-speed image data base 44, a diagnostic information data base 45, an attendant information data base 46 and a program file 47.

The transmission/reception control means 41 functions as both a data sending means and a data receiving means, and controls data receiving from the diagnostic clients 10 and the management client 20 by way of the network 30 and data sending to the diagnostic clients 10 and the management client 20 by way of the network 30.

The controller 42 comprises, for instance, a CPU and executes data transfer, programs and the like.

The high-speed image data base 43 and the low-speed image data base 44 are image storage means which store image data such as image data to be examined.

The high-speed image data base 43 is a memory such as a hard disc and is connected directly to the controller 42 so that image data can be called therefrom at a high speed. In the high-speed image data base 43, pieces of image data representing images which are more expected to be required by the clients such as those representing images which are newly taken and diagnosis on the basis of which is not made yet or those representing past images of the object the images of which are newly taken and diagnosis on the basis of which is expected to be made soon are stored.

The low-speed image data base 44 is a mass storage memory such as a library and is indirectly connected to the controller 42 by way of the high-speed image data base 43. The low-speed image data base 44 receives image data to be stored long from the high-speed image data base 43 and stores it. For example, image data representing images diagnosis on the basis of which has been made or images older than a predetermined time are stored in the low-speed image data base 44. Image data stored in the low-speed image data base can be called at a low speed.

In the diagnostic image data base 45, there are stored, data on the doctor, weights determined by doctor and/or result of examination in liaison with the image data to be examined. That is, the diagnostic image data base 45 includes a result storage means which stores result of examination and stores other various pieces of related information.

The attendant information data base 46 stores information attendant on the image data stored in the high-speed image data base 43 and the low-speed image data base 44. For example, the patient chart, photographing conditions (e.g., photographed part of the body or photographing time) and/or the like is stored in the attendant information data base 46.

Various programs used in the medical image reading system of this embodiment such as a program for controlling the screen of the monitor of the diagnostic clients 10 and the management client 20, a control program for controlling the data input into the controller 42 and the like are stored in the program file 47.

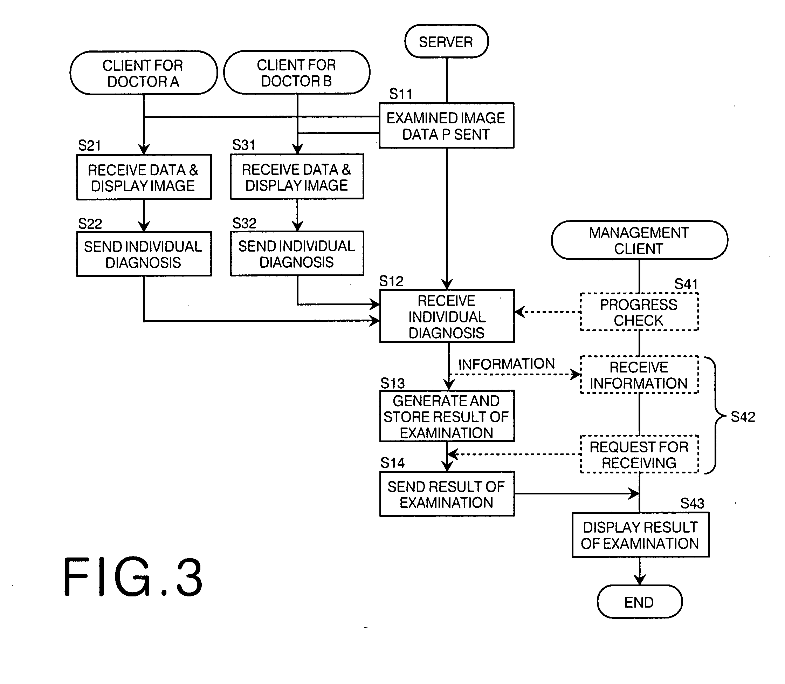

The operation of the medical image reading system of this embodiment will be described with reference to the flow charts shown in Figure 3, hereinbelow.

When examined image data P (medical image data to be examined) is generated and is stored in the high-speed image data base 43, the controller 42 which keeps watching the high-speed image data base 43 sends information to the effect that examined image data P has been stored in the diagnostic information data base 45. This information is stored in the diagnostic information data base 45 as a status of examined image data P "diagnosis waiting". Further codes of doctors A and B in charge of examination of the examined image data P are stored in the diagnostic information data base 45 as data on the doctor in charge of examination.

When doctor A sends a diagnosis initiation signal to the server 40 through the network 30 from his or her diagnostic client 10, the server 40 recognizes that the diagnosis initiation signal is from doctor A on the basis of the signal and confirms that doctor A is in charge of examination of the examined image data P referring to data on the doctor in charge of respective pieces of image data stored in the diagnostic information data base 45. Thereafter, the server 40 retrieves the examined image data P from the high-speed image data base 43 and sends the examined image data P to the diagnostic client 10 for doctor A. (step S11) For the purpose of simplification, doctors A and B are respectively in charge of only one piece of image data in this embodiment.

The diagnostic client 10 for doctor A receives the examined image data P and causes its monitor to display an image represented by the examined image data P. (step S21) Also doctor B sends a diagnosis initiation signal to the server 40 through the network 30 from his or her diagnostic client 10, and the diagnostic client 10 for doctor B receives the examined image data P and causes its monitor to display an image represented by the examined image data P. (step S31)

Doctors A and B separately read the image displayed on the monitor and make diagnosis on the basis of the image. Then doctors A and B sends individual diagnoses to the server 40 through their respective clients 10. (steps S22 and S32) The individual diagnosis may be divided into, for instance, 1:normal and 2:to be reexamined and may be variously divided.

The server 40 receives the individual diagnoses from the diagnostic clients 10 for doctors A and B (step S12). Then the server 40 generates result of examination after receiving individual diagnoses from all the diagnostic clients 10 and stores the result of examination in the diagnostic information data base 45. (step S13) In this particular embodiment, the result of examination is not newly made but comprises individual diagnoses as received.

After the result of examination is stored in step S13, the server 40 sends the result of examination to the management client 20 (step S14), and the management client 20 causes its monitor to display the result of examination received (step S43). The supervisor refers to the result of examination displayed on the monitor of the management client 20 and makes a final determination. Since the result of examination comprises individual diagnoses input by the doctors here, for example, when the individual diagnoses of doctors A and B are both "1: normal", the supervisor's final determination is naturally "1: normal". When the diagnosis of doctor A is "1: normal" while the diagnosis of doctor B is "2: to be reexamined", the supervisor makes the final determination by respectively weighting the diagnoses of doctors A and B. For example, when the weight on doctor A is 1 and that on the doctor B is 0.5, the supervisor's final determination is "1: normal". Conversely when the weight on doctor A is 0.5 and that on the doctor B is 1, the supervisor's final determination is "2: to be reexamined". The weights on the respective doctors have been determined according to the doctor's specialties, experiences and/or abilities and have been stored in the diagnostic information data base 45.

It is possible to check progress of reception of the individual diagnoses and/or the result of examination through the management client 20 before receiving individual diagnoses from all the diagnostic clients 10. (step S41)

Further it is possible to arrange the system so that after the server 40 receives individual diagnoses from all the diagnostic clients 10, the server 40 sends information to the effect that the server 40 has received all the individual diagnoses to the management client 20 and the management client 20 sends to the server a request for receiving the result of examination. (step S42) The information to the effect that the server 40 has received all the individual diagnoses may be sent to the management client at any time between step S12 and S14 and may be, for instance, during step S13 or after step S13.

The diagnostic clients 10, the management client 20, the network 30 and the server 40 need not be necessarily as described above in concrete structure but may be variously modified.

Though, in the embodiment described above, each of the doctors is in charge of only one piece of image data for the purpose of simplification, each doctor may be in charge of two or more pieces of image data. When each doctor is in charge of two or more pieces of image data, the sever 40 sends the plurality of pieces of examined image data to the diagnostic client 10, for instance, in the order in which the pieces of image data are stored in the high-speed image data base 43. Otherwise, the server 40 may send information on which pieces of examined image data are to be read by the doctors obtained from the diagnostic information data base 45 to the clients 10 and the clients 10 may cause their monitors to display lists of pieces of image data versus the doctors so that each doctor may determine the order in which the pieces of image data are to be sent.

Though, in the embodiment described above, one diagnostic client 10 is allotted to one doctor, allotment of the clients 10 to the doctors need not be so. For example, one diagnostic client 10 may be shared by a plurality of doctors and each doctor may send the diagnosis initiation signal after he or she inputs a code inherent to him or her.

Though, in the embodiment described above, the result of examination comprises individual diagnoses as they are, it is possible to arrange the system so that the controller 42 automatically make a determination after the server 40 receives individual diagnoses from all the diagnostic clients 10 and the result of the automatic determination may be taken as the result of examination. In this case, for example, the server 40 may automatically make examination on the basis of the input individual diagnoses and weights for the respective doctors stored in the diagnostic information data base, and the result of the automatic examination may be taken as the result of examination. The program of the automatic examination is stored in the program file 47 and is executed under the control of the controller 42.

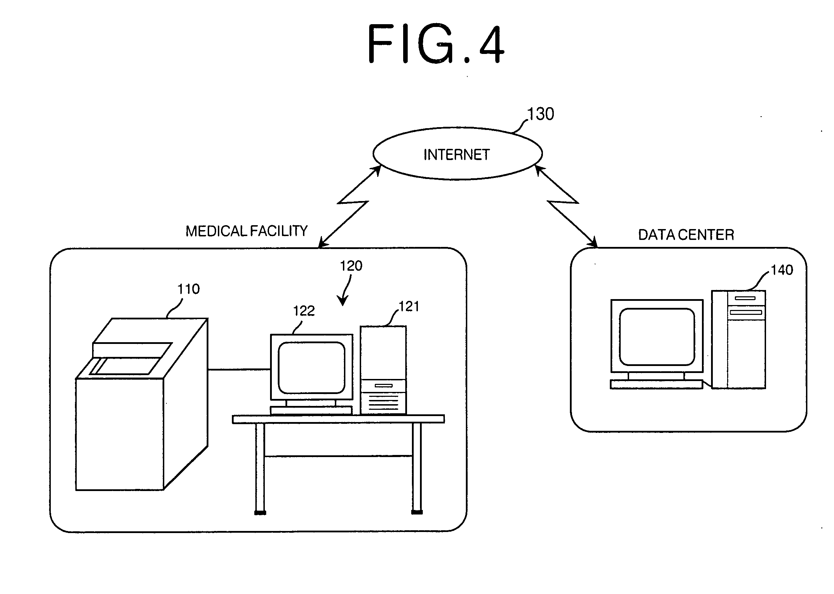

A second embodiment of the present invention which embodies a centralized medical image storing system or a medical information output system in accordance with the present invention will be described with reference to Figures 4 to 6, hereinbelow.

A centralized medical image storing system or a medical information output system (will be simply referred to as the "centralized medical image storing system", hereinbelow) in accordance with the second embodiment of the present invention comprises a digital image data generating apparatus (a CR apparatus) 110 and a client 120 equipped in a medical facility. The client 120 is connected by way of an internet 130 to a server 140 equipped in a data center.

The digital image data generating apparatus 110 is for generating image data representing an image taken in the medical facility, and, for instance, reads a radiation image recorded on a stimulable phosphor sheet by exposing the stimulable phosphor sheet to a radiation passing though an object and digitizes an image signal representing the radiation image. The digital image data generating apparatus 110 is rented to the medical facility from the data center.

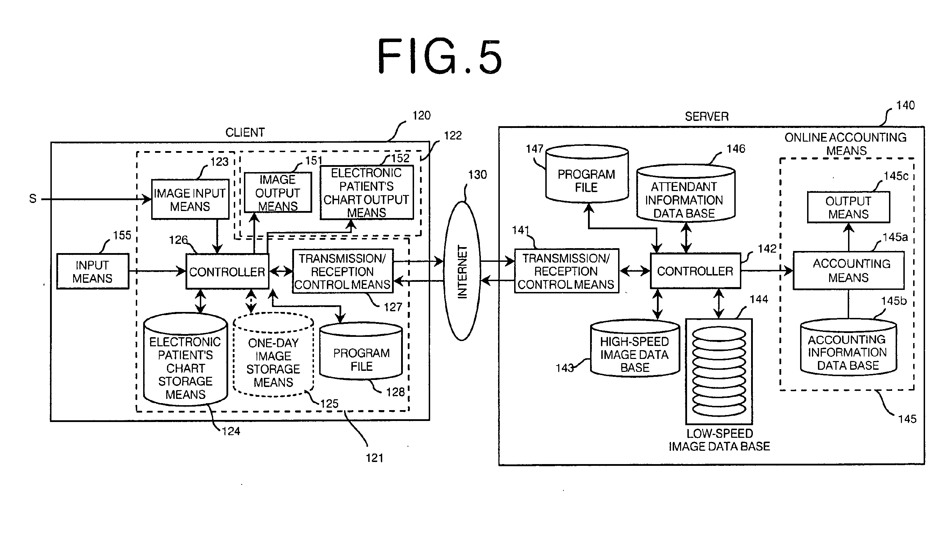

The client 120 comprises a main body 121 provided with a hard disc, a CPU and the like and an input means 155 (Figure 5) such as a keyboard, mouse and/or the like, and is connected to internet 130 by way of a modem built in the main body 121.

As shown in Figure 5, the main body 121 comprises an image input means 123 through which image data S is input from the digital image generating apparatus 110, a controller 126 which has a function of carrying out data transfer, execution of programs, image compression, image processing and the like and a function of a liaison output means which causes an electronic patient's chart output means 152 and an image output means 151 to output in liaison with each other, and a transmission/reception control means 127 which controls transmission and/or reception of data by way of the internet 130. In the hard disc built in the main body 121, there are included an electronic patient's chart storage means 124 which stores electronic patient's charts and a program file 128 which stores programs. In the program file 128, there are stored a display program for an internet web browser to be displayed on a monitor 122, a program for electronic patient's chart creation software which operates on the internet web browser, control programs for various processes to be executed by the controller 126, and the like.

The monitor 122 doubles an image output means 151 and an electronic patient's chart output means 152. That is, the monitor 122 includes an image output means 151 which outputs image data by the use of the internet web browser and an electronic patient's chart output means 152 which outputs electronic patient's charts by the use of the internet web browser, and outputs the image data and the electronic patient's charts as visible images. The monitor 122 is able to output a web browser screen outputting image data sent from the server 140 by way of the internet 130 and a web browser screen outputting electronic patient's charts stored in the electronic patient's chart storage means 124 simultaneously with or separately from each other.

The input means 155 means a mouse, a keyboard and/or the like as described above, and is used in various kinds of input such as input of a request for receiving medical image data, creation of an electronic patient's chart, and recording a result of diagnosis in an electronic patient's chart.

The server 140 comprises a transmission/reception control means 141, a controller 142, a high-speed image data base 143, a low-speed image data base 144, an online accounting means 145, an attendant information data base 146 and a program file 147.

The transmission/reception control means 141 functions as both a data sending means and a data receiving means, and controls data receiving from the clients 120 by way of the internet 130 and data sending to the clients 120 by way of the internet 130.

The controller 142 controls data transfer, execution of programs, compression and expansion of image data and processing transfer to the online accounting means 145.

The high-speed image data base 143 and the low-speed image data base 144 are image storage means which store image data and their storage spaces are divided by medical facilities which have contracted with the data center to use the data bases 143 and 144. The high-speed image data base 143 is a memory such as a hard disc and image data can be called therefrom at a high speed. In the high-speed image data base 143, pieces of image data representing images which are more expected to be required by the clients 120. Image data is compressed according to the purpose thereof since the capacity of the high-speed image data base 143 is limited though relatively large. For example, image data which represents an image for diagnosis and is sent immediately after the image is taken is stored in the high-speed image data base 134 reversibly compressed at a relatively low rate of compression while image data representing an image after diagnosis is stored in the high-speed image data base 134 irreversibly compressed at a higher rate of compression. The low-speed image data base 144 is a mass storage memory such as a library and stores image data to be stored long.

The online accounting means 145 comprises an accounting means 145a, an accounting information data base 145b and an output means 145c. The accounting means 145a calculates accounting information by the medical facilities, e.g., storage cost including, for instance, a charge for using the storage space, a charge for maintenance of medical image data stored in the image data bases 143 and 144, and a service charge for deleting medical image data stored in the image data bases 143 and 144 and stores the accounting information in the accounting information data base 145b or periodically causes the output means (e.g., a printer) 145c to output the accounting information. The output means 145c need not be limited to a printer but may be various means for outputting accounting information to the medical facility, e.g., those sending accounting information to the client 120 by way of the internet 130.

The attendant information data base 146 stores information attendant on the image data stored in the high-speed image data base 143 and the low-speed image data base 144. For example, the patient chart, photographing conditions (e.g., photographed part of the body or photographing time), the ID of the patient, the status of the diagnosis, information on compression, a reference number of the examination and/or the like are stored in the attendant information data base 146. The "status of the diagnosis" represents a status of image data to be examined and is, for instance, "diagnosis waiting", or "diagnosis ended" recorded by images. The "information on compression" is, for instance, whether the image data is reversibly compressed or irreversibly compressed.

Various programs used in the second embodiment such as a program for controlling the screen of the monitor 122 of the clients 120, a program for retrieving image data from the image data bases 143 and 144, and the like are stored in the program file 147.

The operation of the system of this embodiment will be described, hereinbelow.

### 1. Storage of Image Data

Image data S obtained by the digital image data generating apparatus 110 in the medical facility is input into the client 120 by the image input means 123 and then input into the controller 126. The controller 126 sends the image data S to the sever 140 by way of the transmission/reception control means 127 and the internet 130. At this time, since the image data S represents an image on the basis of which a diagnosis is to be made, the controller 126 carries out reversible compression, which preserves image quality, on the image data S before sending the image data S to the server 140.

In the server 140, the transmission/reception control means 141 receives the image data S and stores the image data S in the high-speed image data base 143 by way of the controller 142. At the same time, information to the effect that the image data S has been stored in the high-speed image data base 143 is input into the accounting means 145a together with information representing the size of the image data S from the controller 142. The accounting means 145a calculates the charge for using the storage space according to the size of the image data S and stores it in the accounting information data base 145b. Though the storage space fee (charge for using the storage space) may be constant by the storage space rented to the medical facility irrespective of the image size actually stored or may be changed by the image sizes actually stored. In this particular embodiment, the storage space fee differs by the image sizes actually stored.

### 2. Use of Image Data

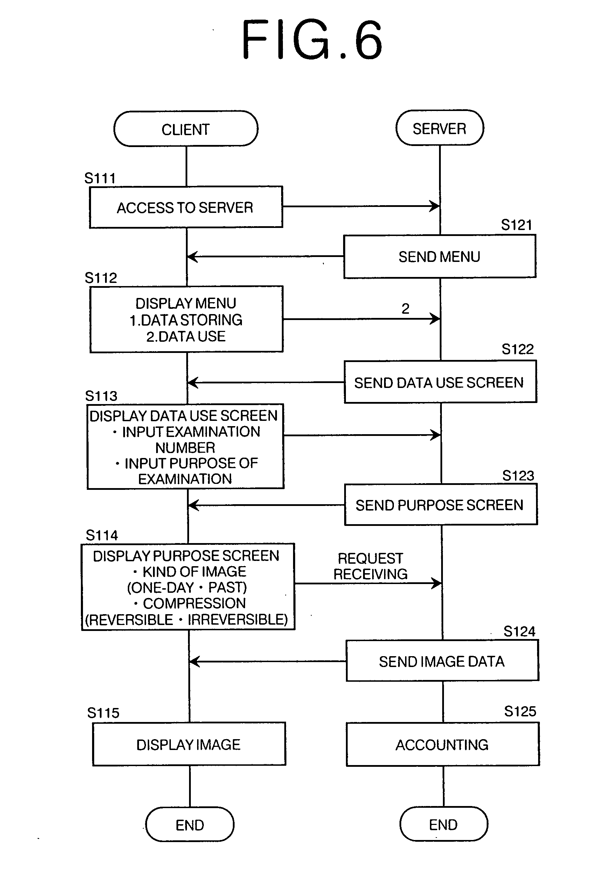

When a doctor or the like uses a piece of image data out of image data stored in the server 140 in order to make a diagnosis on the basis of the piece of image data, he or she can gets the desired piece of image data by accessing the server 140 by way of the internet 130 from the client 120. The processing of using image data in the system of this embodiment will be described with reference to Figure 6, hereinbelow.

When the user (doctor) accesses the server 140 (step S111), the server 140 sends menu to the client 120 (step S121). Then the client 120 causes its monitor 122 to display the menu, and the user selects one of a data storing processing and a data use processing (step S112). When "2. data use" is selected, the server 140 sends a data use screen to the client 120 (step S122) and the client 120 causes its monitor 122 to display the data use screen (step S113).

The data use screen is a screen for inputting a reference number of an examination to be made on the basis of the image data requested, or the purpose of using the image data (e.g., diagnosis, check or reference), and the server 140 sends to the client 120 a purpose screen in which an available range determined related to kinds of images, compression methods and the like is designated. (step S123) That is, the server 140 identifies the patient and usable pieces of image data (e.g., whether there is a one-day image (an image taken on that day), whether there is a past image) with reference to the attendant information data base 146 and sends a purpose screen in which an available range of the compression methods is shown by the identified pieces of image data to the client 120. The available range is determined in order to differently account by purposes of using the image data, and, for instance, when the purpose of using the image data is diagnosis, the available range is set wide but the data fee is set high. To the contrast, when the purpose of using the image data is check, the available range is set narrow but the data fee is set low.

That the purpose of using the image data is diagnosis means that a doctor uses the image data to make a diagnosis, and the available range based on the kinds of image data covers the one-day image (representing the status immediately before diagnosis) and the past image and the available range based on the compression methods (pixel density) covers reversible compression (high density) and irreversible compression (low density). The client 120 causes its monitor to display a purpose screen which is arranged so that the kind of the image, the compression method and/or the like can be selected on the purpose screen. (step S114) That the purpose of using the image data is check means that a doctor uses the image data to check the result of diagnosis, and though the available range based on the kinds of image data covers both the one-day image and the past image, the available range based on the compression methods (pixel density) covers only irreversible compression (low density). That is, in order to check the result of diagnosis, the images need not be so high in pixel density and accordingly the compression method is limited to irreversible compression. That the purpose of using the image data is reference means that a doctor uses the image data only to refer to the images and the result of diagnosis, and accordingly, in this case, the available range based on the kinds of image data covers only the key images (the images which were key in making diagnosis), the available range based on the compression methods (pixel density) covers only irreversible compression (low density). Information on the status of the image data, the compression method and the like can be obtained by referring to the attendant information data base 146.

When a receiving request button is pushed after selecting the kind of the image, the compression method and/or the like on the purpose screen in step S114, the client 120 sends an image receiving request including information on the selected kind of images and the selected compression method to the server 140 and the server 140 sends image data compressed by the compression method designated by the image receiving request. (step S124) The client 120 causes its monitor 122 (image output means 151) to display a visible image on the basis of the image data received. (step S115) When "diagnosis" or "check" is selected as the purpose of using the image data in step S113, desired image processing can be carried on the image data when the monitor 122 displays a visible image on the basis of the image data. That is, for "diagnosis" or "check", it is sometimes preferred that the image data be processed to enhance image quality. Since the image processing to be carried out on the image to enhance image quality can differ by which part of the patient is the object and/or the state of the image, it is preferred that image processing can be carried out or the image processing which the image has been subjected to can be changed. When the purpose of using the image data is "reference", it is preferred that the visible image is displayed on the basis of the image data processed under conditions which have been determined. Whether the client 120 is permitted to carry out image processing is controlled by the server 140.

The server 140 carries out accounting processing after sending the requested image data to the client 120 in step S124. (step S125) That is, the server 140 sums up data fees, which have been determined by purposes for each examination, medical facility by medical facility and stores it in the accounting information data base 145b.

### 3. Use of Electronic Patient's Chart

When an electronic patient's chart is used through the client 120, an electronic patient's chart creation software which operates on the internet web browser is started and the client 120 causes its monitor 122 (the electronic patient's chart output means 152) to display the electronic patient's chart. For example when an electronic patient's chart related to the image displayed is to be used, a chart reference button provided in the browser screen on which the image is displayed is pushed, whereby the controller 126 automatically starts the creation software and electronic patient's charts related to the image displayed are retrieved from the electronic patient's chart storage means 124. The electronic patient's charts thus retrieved are displayed on the electronic chart browser screen of the monitor 122. Each chart stored in the electronic patient's chart storage means 124 is attached with liaison information which relates the patient's chart and the image data with each other, and the controller 126 retrieves the electronic patient's charts with reference to the liaison information. When there is stored no electronic patient's chart related to the image in the electronic patient's chart storage means 124, a blank electronic patient's chart is displayed on the electronic chart browser screen and, for instance, the result of diagnosis is input and a new electronic patient's chart is created.

It is possible to use the electronic patient's chart in other ways. For example, a program for an electronic patient's chart retrieval screen stored in the program file 128 is called through the input means 155 and the monitor 122 (the electronic patient's chart output means 152) is caused to display it. By inputting a predetermined requirement such as the ID of the patient on the screen, the monitor may be caused to display the electronic patient's chart. Further, in order to use image data related to the electronic patient's chart thus displayed, an image reference button provided in the browser screen on which the electronic patient' chart is displayed is pushed, whereby the controller 126 automatically sends, to the server 140, a request for receiving image data related to the electronic patient's chart displayed. An image based on the image data sent from the server 140 in response to the request is displayed on the electronic chart browser screen of the monitor 122.

### 4. Output of Accounting Information

The accounting means 145a of the online accounting means 145 periodically (e.g., monthly) sums accounting information for the respective medical facilities stored in the accounting information data base 145a by items (e.g., the rent, the storage cost, the data fee and the like) and outputs the accounting information (a bill) to the respective medical facilities.

For example, the rent may include a rent (¥A/month) for the digital image generating apparatus 110 rented to the medical facility from the data center.

For example the storage cost may be as follows.

The total for the month of the storage space fee (¥B/image size) : (¥B×i).

The total for the month of the charges for maintenance of medical image data stored in the image storage means (e.g., 1 to 3 years old images: ¥C/image, 3 to 5 years old images: ¥D/image, 5 to 10 years old images: ¥E/image): ¥(xC+yD+zE)

The total for the month of the service charge for deleting medical image data (¥F/image):¥F×j

The data fee is the total for the month of a diagnostic fee (¥G/examination), a check fee (¥H/examination) and a reference fee (¥J/examination):¥(kG+mH+nJ)

That is, the total for the month (A+iB+xC+yD+zE+jF+mH+nJ) of the rent, the storage cost, the data fee and the like stored in the accounting information data base 145b is calculated by the accounting means 145a and the total is output by the output means 145c.

Though, in the embodiment described above, also the one-day image representing the status before diagnosis is sent to the server 140 and stored therein, it is possible to provide the hard disc of the client 120 with a one-day image storage means 125 and image data representing one-day images may be stored in the one-day image storage means 125. In this case, the one-day image data is input through the image input means 123 and stored in the one-day image storage means 125 by way of the controller 126, and output as a visible image through the image output means 151 as requested through the input means 155. After diagnosis, the one-day image data is reversibly compressed and sent to the server 140.

With this arrangement, images before diagnosis which should be high in pixel density can be displayed without receiving from the server 140, and accordingly, the time required to receive the image data can be saved. For example, it takes about five minutes to receive a one-day image data for diagnosis (reversibly compressed), and to the contrast, it takes only about twenty to thirty seconds to receive a past image data for reference (irreversibly compressed). (in the case of using ISDN)

Though, in the embodiment described above, an internet is employed as the network, other various kinds of networks may be employed.

Further, though, in the embodiment described above, the storage space fee is charged by images, a predetermined capacity of a storage space may be rent for a fixed charge. In this case, the accounting means accounts the fixed charge as the storage space fee. Further, in this case, it may be possible to charge the storage space fee by images when the storage space occupied by the image data stored by a medical facility exceeds the predetermined capacity.

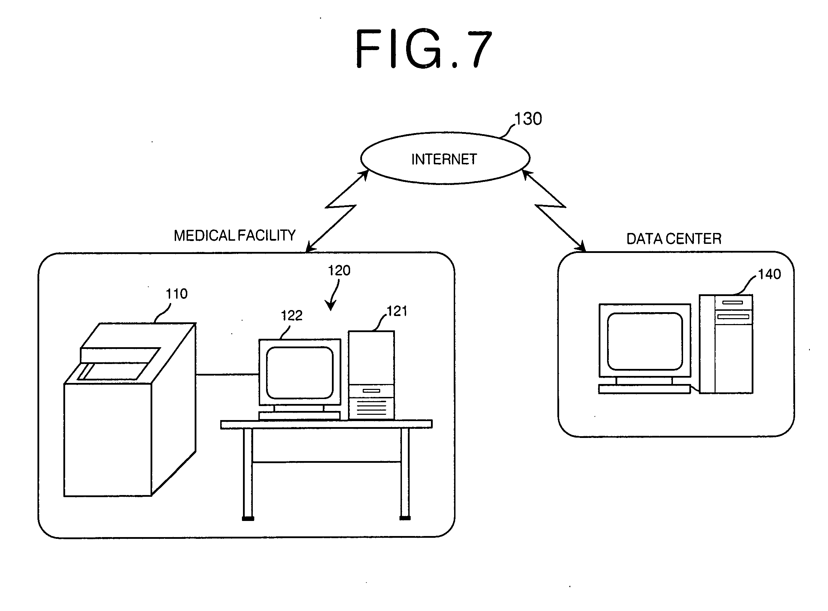

A medical information storing and accounting system in accordance with a third embodiment of the present invention will be described with reference to Figures 7 to 9, hereinbelow. In Figures 7 to 9, elements analogous to those shown in Figures 4 to 6 are given the same reference numerals.

A medical information storing and accounting system in accordance with the third embodiment of the present invention comprises a digital image data generating apparatus (a CR apparatus) 110 and a client 120 equipped in a medical facility. The client 120 is connected by way of an internet 130 to a server 140 equipped in a data center.

The digital image data generating apparatus 110 is for generating image data representing an image taken in the medical facility, and, for instance, reads a radiation image recorded on a stimulable phosphor sheet by exposing the stimulable phosphor sheet to a radiation passing though an object and digitizes an image signal representing the radiation image. The digital image data generating apparatus 110 is rented to the medical facility from the data center.

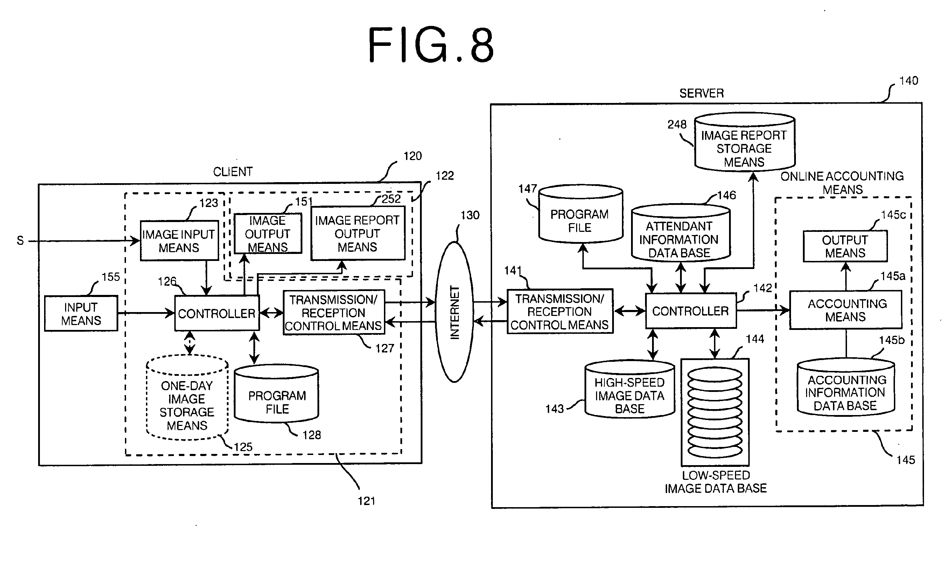

The client 120 comprises a main body 121 provided with a hard disc, a CPU and the like and an input means 155 (Figure 8) such as a keyboard, mouse and/or the like, and is connected to internet 130 by way of a modem built in the main body 121.

As shown in Figure 8, the main body 121 comprises an image input means 123 through which image data S is input from the digital image generating apparatus 110, a controller 126 which has a function of carrying out data transfer, execution of programs, image compression, image processing and the like, and a transmission/reception control means 127 which controls transmission and/or reception of data by way of the internet 130. In the hard disc built in the main body 121, there is included a program file 128 which stores programs. In the program file 128, there are stored a display program for an internet web browser to be displayed on a monitor 122, and control programs for various processes to be executed by the controller 126, and the like.

The monitor 122 doubles an image output means 151 and an image report output means 152. That is, the monitor 122 includes an image output means 151 which outputs image data by the use of the internet web browser and an image report output means 152 which outputs image reports by the use of the internet web browser, and outputs the image data and the image reports as visible images. The monitor 122 is able to output image data sent from the server 140 by way of the internet 130 and image reports.

The input means 155 means a mouse, a keyboard and/or the like as described above, and is used in various kinds of input such as input of a request for receiving medical image data or an image report, creation of an image report, and recording a result of diagnosis in an image report.

The server 140 comprises a transmission/reception control means 141, a controller 142, a high-speed image data base 143, a low-speed image data base 144, an online accounting means 145, an attendant information data base 146, a program file 147 and an image report storage means 248.

The transmission/reception control means 141 functions as both a data sending means and a data receiving means, and controls data receiving from the clients 120 by way of the internet 130 and data sending to the clients 120 by way of the internet 130.

The controller 142 controls data transfer, execution of programs, compression and expansion of image data and processing transfer to the online accounting means 145.

The high-speed image data base 143 and the low-speed image data base 144 are image storage means which store image data and their storage spaces are divided by medical facilities which have contracted with the data center to use the data bases 143 and 144. The high-speed image data base 143 is a memory such as a hard disc and image data can be called therefrom at a high speed. In the high-speed image data base 143, pieces of image data representing images which are more expected to be required by the clients 120. Image data is compressed according to the purpose thereof since the capacity of the high-speed image data base 143 is limited though relatively large. For example, image data which represents an image for diagnosis and is sent immediately after the image is taken is stored in the high-speed image data base 134 reversibly compressed at a relatively low rate of compression while image data representing an image after diagnosis is stored in the high-speed image data base 134 irreversibly compressed at a higher rate of compression. The low-speed image data base 144 is a mass storage memory such as a library and stores image data to be stored long.

The online accounting means 145 comprises an accounting means 145a, an accounting information data base 145b and an output means 145c. The accounting means 145a calculates accounting information by the medical facilities, e.g., storage cost including, for instance, a charge for using the storage space, a charge for maintenance of medical image data stored in the image data bases 143 and 144, and a service charge for deleting medical image data stored in the image data bases 143 and 144 and stores the accounting information in the accounting information data base 145b or periodically causes the output means (e.g., a printer) 145c to output the accounting information. The output means 145c need not be limited to a printer but may be various means for outputting accounting information to the medical facility, e.g., those sending accounting information to the client 120 by way of the internet 130.

The attendant information data base 146 stores information attendant on the image data stored in the high-speed image data base 143 and the low-speed image data base 144. For example, photographing conditions (e.g., photographed part of the body or photographing time), the ID of the patient, the status of the diagnosis, information on compression, a reference number of the examination and/or the like are stored in the attendant information data base 146. The "status of the diagnosis" represents a status of image data to be examined and is, for instance, "diagnosis waiting", or "diagnosis ended" recorded by images. The "information on compression" is, for instance, whether the image data is reversibly compressed or irreversibly compressed.

Various programs used in the third embodiment such as a program for controlling the screen of the monitor 122 of the clients 120, a program for retrieving image data from the image data bases 143 and 144, various control programs to be executed by the controller 142 and the like are stored in the program file 147.

In the image report storage means 248, there are stored image reports. The image data includes information for identifying image data related thereto.

The operation of the system of this embodiment will be described, hereinbelow.

### 1. Storage of Image Data

Image data S obtained by the digital image data generating apparatus 110 in the medical facility is input into the client 120 by the image input means 123 and then input into the controller 126. The controller 126 sends the image data S to the sever 140 by way of the transmission/reception control means 127 and the internet 130. At this time, since the image data S represents an image on the basis of which a diagnosis is to be made, the controller 126 carries out reversible compression, which preserves image quality, on the image data S before sending the image data S to the server 140.

In the server 140, the transmission/reception control means 141 receives the image data S and stores the image data S in the high-speed image data base 143 by way of the controller 142. At the same time, information to the effect that the image data S has been stored in the high-speed image data base 143 is input into the accounting means 145a together with information representing the size of the image data S from the controller 142. The accounting means 145a calculates the charge for using the storage space according to the size of the image data S and stores it in the accounting information data base 145b. Though the storage space fee (charge for using the storage space) may be constant by the storage space rented to the medical facility irrespective of the image size actually stored or may be changed by the image sizes actually stored. In this particular embodiment, the storage space fee differs by the image sizes actually stored.

### 2. Use of Image Data

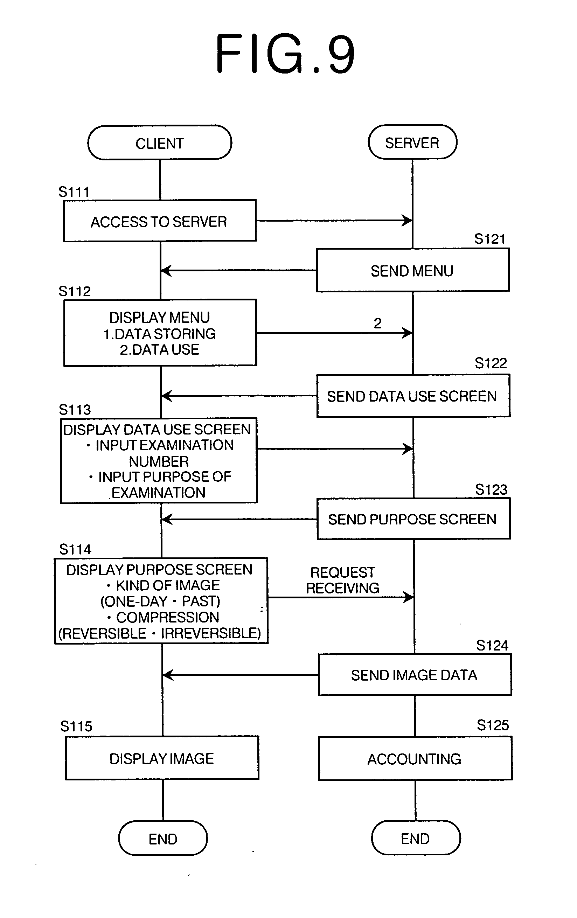

When a doctor or the like uses a piece of image data out of image data stored in the server 140 in order to make a diagnosis on the basis of the piece of image data, he or she can gets the desired piece of image data by accessing the server 140 by way of the internet 130 from the client 120. The processing of using image data in the system of this embodiment will be described with reference to Figure 9, hereinbelow.

When the user (doctor) accesses the server 140 (step S111), the server 140 sends menu to the client 120 (step S121). Then the client 120 causes its monitor 122 to display the menu, and the user selects one of a data storing processing and a data use processing (step S112). When "2. data use" is selected, the server 140 sends a data use screen to the client 120 (step S122) and the client 120 causes its monitor 122 to display the data use screen (step S113).

The data use screen is a screen for inputting a reference number of an examination to be made on the basis of the image data requested, or the purpose of using the image data (e.g., diagnosis, check or reference), and the server 140 sends to the client 120 a purpose screen in which an available range determined related to kinds of images, compression methods and the like is designated. (step S123) That is, the server 140 identifies the patient and usable pieces of image data (e.g., whether there is a one-day image (an image taken on that day), whether there is a past image) with reference to the attendant information data base 146 and sends a purpose screen in which an available range of the compression methods is shown by the identified pieces of image data to the client 120. The available range is determined in order to differently account by purposes of using the image data, and, for instance, when the purpose of using the image data is diagnosis, the available range is set wide but the data fee is set high. To the contrast, when the purpose of using the image data is check, the available range is set narrow but the data fee is set low.

That the purpose of using the image data is diagnosis means that a doctor uses the image data to make a diagnosis, and the available range based on the kinds of image data covers the one-day image (representing the status immediately before diagnosis) and the past image and the available range based on the compression methods (pixel density) covers reversible compression (high density) and irreversible compression (low density). The client 120 causes its monitor to display a purpose screen which is arranged so that the kind of the image, the compression method and/or the like can be selected on the purpose screen. (step S114) That the purpose of using the image data is check means that a doctor uses the image data to check the result of diagnosis, and though the available range based on the kinds of image data covers both the one-day image and the past image, the available range based on the compression methods (pixel density) covers only irreversible compression (low density). That is, in order to check the result of diagnosis, the images need not be so high in pixel density and accordingly the compression method is limited to irreversible compression. That the purpose of using the image data is reference means that a doctor uses the image data only to refer to the images and the result of diagnosis, and accordingly, in this case, the available range based on the kinds of image data covers only the key images (the images which were key in making diagnosis), the available range based on the compression methods (pixel density) covers only irreversible compression (low density). Information on the status of the image data, the compression method and the like can be obtained by referring to the attendant information data base 146.

When a receiving request button is pushed after selecting the kind of the image, the compression method and/or the like on the purpose screen in step S114, the client 120 sends an image receiving request including information on the selected kind of images and the selected compression method to the server 140 and the server 140 sends image data compressed by the compression method designated by the image receiving request. (step S124) The client 120 causes its monitor 122 (image output means 151) to display a visible image on the basis of the image data received. (step S115) When "diagnosis" or "check" is selected as the purpose of using the image data in step S113, desired image processing can be carried on the image data when the monitor 122 displays a visible image on the basis of the image data. That is, for "diagnosis" or "check", it is sometimes preferred that the image data be processed to enhance image quality. Since the image processing to be carried out on the image to enhance image quality can differ by which part of the patient is the object and/or the state of the image, it is preferred that image processing can be carried out or the image processing which the image has been subjected to can be changed. When the purpose of using the image data is "reference", it is preferred that the visible image is displayed on the basis of the image data processed under conditions which have been determined. Whether the client 120 is permitted to carry out image processing is controlled by the server 140.

When "diagnosis" is selected as the purpose of using the image data in step S113, the client 120 is permitted to receive image data related to the examination, to cause its monitor to display a visible image on the basis of the image data and to input. When "check" or "reference" is selected as the purpose of using the image data in step S113, the client 120 is permitted only to cause its monitor to display related image reports. When a report reference button provided in the browser screen on which the image is displayed is pushed, image reports related to the image displayed are retrieved from the image report storage means 248 and are displayed on the electronic chart browser screen of the monitor 122. When there is stored no image report related to the image in the image report storage means 248, a blank image report is displayed on the image report browser screen and, for instance, the result of diagnosis is input and a new image report is created.

The server 140 carries out accounting processing after sending the requested image data to the client 120 in step S124. (step S125) That is, the server 140 sums up data fees, which have been determined by purposes for each examination, medical facility by medical facility and stores it in the accounting information data base 145b.

### 3. Output of Accounting Information

The accounting means 145a of the online accounting means 145 periodically (e.g., monthly) sums accounting information for the respective medical facilities stored in the accounting information data base 145a by items (e.g., the rent, the storage cost, the data fee and the like) and outputs the accounting information (a bill) to the respective medical facilities.

For example, the rent may include a rent (¥A/month) for the digital image generating apparatus 110 rented to the medical facility from the data center.

For example the storage cost may be as follows.

The total for the month of the storage space fee (¥B/image size) : (¥B×i).

The total for the month of the charges for maintenance of medical image data stored in the image storage means (e.g., 1 to 3 years old images: ¥C/image, 3 to 5 years old images: ¥D/image, 5 to 10 years old images: ¥E/image): ¥(xC+yD+zE)

The total for the month of the service charge for deleting medical image data (¥F/image):¥F×j

The data fee is the total for the month of a diagnostic fee (¥G/examination), a check fee (¥H/examination) and a reference fee (¥J/examination):¥(kG+mH+nJ)

That is, the total for the month (A+iB+xC+yD+zE+jF+mH+nJ) of the rent, the storage cost, the data fee and the like stored in the accounting information data base 145b is calculated by the accounting means 145a and the total is output by the output means 145c.

Though, in the embodiment described above, also the one-day image representing the status before diagnosis is sent to the server 140 and stored therein, it is possible to provide the hard disc of the client 120 with a one-day image storage means 125 and image data representing one-day images may be stored in the one-day image storage means 125. In this case, the one-day image data is input through the image input means 123 and stored in the one-day image storage means 125 by way of the controller 126, and output as a visible image through the image output means 151 as requested through the input means 155. After diagnosis, the one-day image data is reversibly compressed and sent to the server 140.

With this arrangement, images before diagnosis which should be high in pixel density can be displayed without receiving from the server 140, and accordingly, the time required to receive the image data can be saved. For example, it takes about five minutes to receive a one-day image data for diagnosis (reversibly compressed), and to the contrast, it takes only about twenty to thirty seconds to receive a past image data for reference (irreversibly compressed). (in the case of using ISDN)

Though, in the embodiment described above, an internet is employed as the network, other various kinds of networks may be employed.

Further, though, in the embodiment described above, the storage space fee is charged by images, a predetermined capacity of a storage space may be rent for a fixed charge. In this case, the accounting means accounts the fixed charge as the storage space fee. Further, in this case, it may be possible to charge the storage space fee by images when the storage space occupied by the image data stored by a medical facility exceeds the predetermined capacity.

## Claims

1. A medical image reading system comprising
a plurality of diagnostic clients provided with an image output means which outputs image data to be examined as a visible image, and a diagnosis input means for inputting individual diagnoses obtained on the basis of visible images,
at least one management client provided with a result output means for outputting result of examination obtained on the basis of the individual diagnoses, and
a server provided with an image storage means and a result storage means which are connected to the diagnostic clients and the management client by way of a network, the image storage means storing image data to be examined and the result storage means storing results of examination obtained on the basis of the individual diagnoses with the results of examination related to the image data, wherein
each of the diagnostic clients receives the image data to be examined from the server by way of a network, outputs the image data to be examined through the image output means and sends individual diagnoses input through the diagnosis input means for the respective images represented by the image data to be examined to the server by way of the network,
the server causes the result storage means to store results of examination obtained on the basis of the individual diagnoses sent from the respective diagnostic clients, and
the management client receives the result of examination from the server by way of the network and causes the result output means to output the same.

2. A medical image reading system as defined in Claim 1 in which said server is further provided with an informing means which, when the server receives a predetermined number of said diagnoses, sends information to the effect that the server has received a predetermined number of said diagnoses to the management client and the management client is provided with an information output means which receives the information and outputs the same.

3. A medical image reading system as defined in Claim 1 in which said server further comprises an automatic determination means which automatically makes examination for a given piece of image data on the basis of all or part of the individual diagnoses received and outputs result of examination.

4. A medical image reading system as defined in Claim 3 in which, each of the diagnostic clients is provided with a function of sending data on the doctor in charge together with the relevant individual diagnosis to the server so that the automatic determination means makes examination weighting the individual diagnoses according to the doctor in charge.

5. A diagnostic client for a medical image reading system defined in Claim 1 which client is connected by way of a network to a server provided with an image storage means which stores image data to be examined and a result storage means which stores results of examination obtained on the basis of the individual diagnoses, and comprises
a data receiving means which receives the image data to be examined from the server,
an image output means which outputs the image data to be examined as a visible image,
a diagnosis input means for inputting individual diagnoses obtained on the basis of visible images, and
a data sending means which sends the individual diagnoses input through the diagnosis input means to the server.

6. A diagnostic client as defined in Claim 5 in which said data sending means sends data on the doctor in charge together with the relevant individual diagnosis to the server.

7. A management client for a medical image reading system defined in Claim 1 which client is connected by way of a network to a server provided with an image storage means which stores image data to be examined and a result storage means which stores results of examination obtained on the basis of the individual diagnoses, and comprises
a data receiving means which receives results of examination from the server, and
a result output means which outputs the results of examination received by the data receiving means.

8. The management client as defined in Claim 7 in which
said data receiving means is further provided with an information output means which receives information to the effect that the server has received a predetermined number of said diagnoses from the server and outputs the same.

9. A server for a medical image reading system defined in Claim 1 which server is connected by way of a network to a plurality of diagnostic client and at least one management client and comprises
an image storage means which stores image data to be examined,
a data sending means which sends the image data stored in the image storage means to the diagnostic clients,
a data receiving means which receives from the diagnostic clients individual diagnoses obtained on the basis of the image data to be examined, and
a result storage means which stores results of examination obtained on the basis of the individual diagnoses with the results of examination related to the image data to be examined, wherein
said data sending means is further provided with a function of sending to the management clients the results of examination stored in the result storage means.

10. A server as defined in Claim 9 in which said data sending means is further provided an information sending means which sends to the management client information to the effect that the server has received a predetermined number of said diagnoses.

11. A server as defined in Claim 9 in which said data sending means is further provided with an automatic determination means which automatically makes examination for a given piece of image data on the basis of all or part of the individual diagnoses received and outputs result of examination.

12. A server as defined in Claim 11 in which said data receiving means receives data on the doctor in charge together with the relevant individual diagnosis from the diagnostic client, and
the automatic determination means makes examination weighting the individual diagnoses according to the doctor in charge.

13. A program for causing a computer to execute
processing of sending image data to be examined stored in an image storage means to a diagnostic client,
processing of receiving individual diagnoses obtained on the basis of the image data to be examined sent from the diagnostic client,
processing of storing in a result storage means results of examination obtained on the basis of the individual diagnoses with the results of examination related to the image data to be examined, and
processing of sending the results of examination stored in the result storage means to the management client.

14. A program as defined in Claim 13 for causing a computer to further execute processing of sending to the management client information to the effect that a predetermined number of said diagnoses has been input.

15. A program as defined in Claim 13 for causing a computer to further execute processing of automatically making examination for a given piece of image data on the basis of all or part of the individual diagnoses received.

16. A program as defined in Claim 15 in which
the automatic examination is made weighting the individual diagnoses on the basis of data on the doctor in charge sent from the diagnostic client together with the individual diagnoses.

17. A medical image reading method comprising the steps of
storing image data to be examined in a server which is provided at a place remote from a plurality of diagnostic clients and connected to the diagnostic clients by way of a network,
causing each of the diagnostic clients to receive a desired piece of image data out of the image data stored in the server and to output the desired piece of image data as a visible image,
inputting individual diagnoses obtained on the basis of the output visible image through the diagnostic clients,
sending the individual diagnoses to the server,
storing in the server results of examination obtained on the basis of the individual diagnoses sent thereto with the results of examination related to the pieces of image data, and
causing a management client to receive the result of examination for a desired piece of image data stored in the server and to output the same.

18. A medical image reading method as defined in Claim 17 further comprising the step of
causing the server, when the server receives all the diagnoses, to send information to the effect that the server has received all of said diagnoses to the management client.

19. A medical image reading method as defined in Claim 17 in which the result of examination is weighted by individual diagnoses..

20. A centralized medical image storing system comprising
a plurality of clients each of which is provided in a medical facility, provided with an image input means for inputting medical image data obtained by a digital image data generating apparatus, and transfers medical image data by way of a network, and
a server which is connected to the clients by way of the network and provided with an image storage means and an online accounting means, the image storage means storing medical image data sent from the clients and the online accounting means automatically outputting accounting information for medical facilities,
wherein the improvement comprises that
each of the clients is provided with a function of sending a request for receiving a desired piece of medical image data out of image data stored in the image storage means to the server, the server is provided with a function of sending the desired piece of image data to the client which sends the request in response to receipt of the request, and each of the clients is provided with an image output means which outputs as a visible image the desired image data sent from the server.

21. A centralized medical image storing system as defined in Claim 20 in which the image output means further outputs as a visible image the medical image data input through the image input means.

22. A centralized medical image storing system as defined in Claim 20 in which the accounting information includes storage cost for the medical image data which the medical facility holds in possession.

23. A centralized medical image storing system as defined in Claim 20 in which the digital image generating apparatus and the client are rented to the medical facility and the accounting information includes a rent for the digital image generating apparatus and the client.

24. A centralized medical image storing system as defined in Claim 20 in which the accounting information includes a data fee imposed on the medical image data used through the client.

25. A centralized medical image storing system as defined in Claim 20 in which the storage means comprises a high-speed storage means and a low-speed storage mean and the high-speed storage means can transfer the medical image data stored therein to the client at a high speed while the low-speed storage means can transfer the medical image data stored therein to the client at a low speed.

26. A client for a centralized medical image storing system defined in Claim 20 comprising
an image input means for inputting medical image data obtained by a digital image data generating apparatus,
a first data sending means which transfers the medical image data input through the image input means to a sever by way of a network,
a second data sending means which sends a request for receiving a desired piece of medical image data out of image data stored in the server to the server,
a data receiving means which receives from the sever the desired piece of medical image data as based on the request sent from the second data transfer means, and
an image output means which outputs as a visible image the desired piece of medical image data received by the data receiving means.

27. A client as defined in Claim 26 in which the image output means further outputs as a visible image the medical image data input through the image input means.

28. A server for a centralized medical image storing system defined in Claim 20 comprising
a first data receiving means which receives medical image data from a plurality of clients by way of a network,
a storage means which stores the medical image data received by the first data receiving means,
a second data receiving means which receives a request for receiving a desired piece of medical image data out of image data stored in the server from the clients,
a data sending means which sends to the clients the desired piece of medical image data as based on the request received by the second data receiving means, and
an online accounting means which automatically outputs accounting information for medical facilities equipped with the clients.

29. A server as defined in Claim 28 in which the accounting information includes storage cost for the medical image data which the medical facility holds in possession.

30. A server as defined in Claim 28 in which the digital image generating apparatus and the client are rented to the medical facility and the accounting information includes a rent for the digital image generating apparatus and the client.

31. A server as defined in Claim 28 in which the accounting information includes a data fee imposed on the medical image data used through the client.

32. A server as defined in Claim 28 in which the storage means comprises a high-speed storage means and a low-speed storage mean and the high-speed storage means can transfer the medical image data stored therein to the client at a high speed while the low-speed storage means can transfer the medical image data stored therein to the client at a low speed.

33. A program for causing a computer to execute
processing of sending medical image data to a sever by way of a network,
processing of sending a request for receiving a desired piece of medical image data out of image data stored in the server to the server, and
processing of outputting as a visible image the desired piece of medical image data.

34. A program for causing a computer to execute
processing of receiving medical image data from a plurality of clients by way of a network,
processing of storing the medical image data received,
processing of receiving a request for receiving a desired piece of medical image data out of image data stored in the server from the clients,
processing of sending to the clients the desired piece of medical image data as based on the request received, and
processing of automatically outputting accounting information for medical facilities equipped with the clients.

35. A centralized medical image storing method comprising the steps of
inputting medical image data obtained by a digital image data generating apparatus through a plurality of clients each of which is provided in a medical facility, transferring the medical image data to a server by way of a network,
storing the medical image data in the server,
causing the clients to send a request for receiving a desired piece of medical image data out of image data stored in the server to the server,
causing the server to send the desired piece of image data to the client which sends the request in response to receipt of the request,
causing the clients to output as a visible image the desired image data sent from the server, and
causing the server to automatically output accounting information for medical facilities.

36. A method as defined in Claim 35 in which the accounting information includes storage cost for the medical image data which the medical facility holds in possession and is stored in the server.

37. A method as defined in Claim 35 in which the digital image generating apparatus and/or the client are rented to the medical facility and the accounting information includes a rent for the digital image generating apparatus and/or the client.

38. A method as defined in Claim 35 in which the accounting information includes a data fee imposed on the medical image data which is stored in the server and used through the client.

39. A medical information storing and accounting system comprising
a plurality of clients each of which is provided in a medical facility, receives medical image data by way of a network and is provided with an image output means which outputs as a visible image the medical image data received, and
a server which is connected to the clients by way of the network and provided with an image storage means which stores medical image data and an online accounting means which automatically outputs accounting information for medical facilities,
wherein the improvement comprises that
each of the clients is provided with a function of sending a request for receiving medical image data related to a predetermined examination out of image data stored in the image storage means to the server,
the server is provided with a function of sending, to the client which sends the request, image data as requested by the client, and
the accounting information output by the online accounting means includes a data fee determined examination by examination.

40. A medical information storing and accounting system as defined in Claim 39 in which each of the clients is arranged to send, to the server, purpose information representing the purpose of examination together with the request for receiving medical image data related to the predetermined examination, and
the server is arranged to send to the client medical image data related to the predetermined examination within an available range determined purpose by purpose on the basis of the purpose information with the data fee determined by purpose of examination.

41. A medical information storing and accounting system as defined in Claim 39 in which the available range is a range related to one or more of the kind of images, the pixel density of images and image processing right.

42. A medical information storing and accounting system as defined in Claim 39 in which
the server further comprises a report storage means which stores a report on the medical image data,
each of the clients further comprises a report output means which receives a report stored in the report storage means and outputs it, and
the server sends, to the client, image data as requested by the client together with the report on the medical image data.

43. A medical information storing and accounting system as defined in Claim 39 in which
the server further comprise a report storage means which stores a report on the medical image data,
each of the clients further comprises a report output means which receives a report stored in the report storage means and outputs it, a report input means for inputting a report and a function of sending, to the sever, the report input through the report input means,
the server sends, to the client, image data as requested by the client together with the report on the medical image data, and
the available range is a range related to one or more of the kind of images, the pixel density of images, image processing right and report input right.

44. A client for a medical information storing and accounting system defined in Claim 39 comprising
a data sending means which sends a request for receiving medical image data related to a predetermined examination out of image data stored in a server connected thereto by way of a network,
a data receiving means which receives, from the server, medical image data as requested by the request for receiving medical image data sent by the data sending means, and
an image output means which outputs as a visible image the medical image data received by the data receiving means.

45. A client as defined in Claim 44 in which the data sending means sends, to the server, purpose information representing the purpose of examination together with the request for receiving medical image data related to the predetermined examination, and
the data receiving means receives image data related to the predetermined examination within an available range determined purpose by purpose on the basis of the purpose information.

46. A client as defined in Claim 44 further comprising a second data receiving means which receives a report on the medical image data stored in the server, and
a report output means which outputs the report received by the second data receiving means.

47. A client as defined Claim 44 further comprising a report input means for inputting said report and
a function of sending, to the sever, the report input through the report input means.

48. A server for a medical information storing and accounting system defined in Claim 39 comprising
an image storage means which stores medical image data,
a data receiving means which receives a request for receiving medical image data related to a predetermined examination out of image data stored in the image storage means from a client connected thereto by way of a network,
a data sending means which sends, to the client, medical image data as requested by the request for receiving medical image data received by the data receiving means, and
an online accounting means which automatically outputs accounting information for the medical facility equipped with the client, the accounting information including a data fee determined examination by examination.

49. A server as defined in Claim 48 in which the data sending means sends medical image data within an available range determined purpose by purpose on the basis of the purpose information which represents the purpose of examination and is sent from the client together with the request for receiving medical image data related to the predetermined examination with the data fee determined by purpose of examination.

50. A server as defined in Claim 49 in which the available range is a range related to one or more of the kind of images, the pixel density of images and image processing right.

51. A server as defined in Claim 49 which further comprises a report storage means which stores a report on the medical image data, and
sends, to the client, image data as requested by the request for receiving medical image data together with the report on the medical image data.

52. A sever as defined in Claim 49 which further comprises a report storage means which stores a report on the medical image data, and sends, to the client, image data as requested by the request for receiving medical image data together with the report on the medical image data, and in which the available range is a range related to one or more of the kind of images, the pixel density of images, image processing right and report input right.

53. A program for causing a computer to execute
processing of sending a request for receiving medical image data related to a predetermined examination out of image data stored in a sever by way of a network to the server,
processing of receiving medical image data as requested by the request for receiving medical image data, and
processing of outputting as a visible image the medical image data received.

54. A program as defined in Claim 53 for causing a computer to further execute
processing of sending, to the server, purpose information representing the purpose of examination together with the request for receiving medical image data related to the predetermined examination, and
processing of receiving medical image data related to the predetermined examination within an available range determined purpose by purpose on the basis of the purpose information.

55. A program for causing a computer to execute
processing of storing medical image data,
processing of receiving a request for receiving medical image data related to a predetermined examination out of image data stored in the image storage means from a client connected thereto by way of a network,
processing of sending, to the client, medical image data as requested by the request for receiving medical image data received by the data receiving means, and
processing of automatically outputting accounting information for the medical facility equipped with the client, the accounting information including a data fee determined examination by examination.

56. A program as defined in Claim 55 for causing a computer to further execute
processing of sending medical image data related to the predetermined examination within an available range determined purpose by purpose on the basis of purpose information sent from the client together with the request for receiving medical image data, and
processing of determining the data fee examination by examination.

57. A medical information storing and accounting method comprising the steps of
storing image data in a server connected by way of a network to a plurality of clients each of which is provided in a medical facility,
sending, from the server to the client which sends a request for receiving medical image data related to a predetermined examination out of image data stored in the server, image data as requested by the client,
outputting through the client the medical image data sent by the server, and
causing the server to automatically output accounting information for medical facilities, the accounting information including a data fee determined examination by examination.

58. A method as defined in Claim 57 further comprising the steps of
determining an available range purpose by purpose, and
determining the data fee examination by examination and purpose by purpose.

59. A method as defined in Claim 57 further comprising the steps of
storing image reports on the medical image data in the server,
sending, to the client, medical image data as requested by the client together with the report on the medical image data, and
outputting, from the client, medical image data as requested by the client together with the report on the medical image data.

60. A medical information output system comprising
a plurality of clients each of which is equipped in a medical facility and is provided with an image output means which receives medical image data by way of a network and outputs as a visible image the medical image data received, an electronic patient's chart storage means which stores data on the patient's chart, and an electronic patient's chart output means which outputs data on the patient's chart stored in the electronic patient's chart storage means, and
a server which is connected to the clients by way of the network and is provided with an image storage means which stores medical image data, wherein the improvement comprises that
each of the clients is provided with a liaison output means which causes the electronic patient's chart output means and the image output means to output in liaison with each other, and the liaison output means causes the electronic patient's chart output means and the image output means to output data on the patient's chart and medical image data corresponding to the patient's chart by the use of liaison information which relates the patient's chart and the medical image data with each other.

61. A medical information output system as defined in Claim 60 in which
each of the clients is provided with a function of sending a request for receiving medical image data out of image data stored in the image storage means to the server,
the server is provided with a function of sending image data to the client which sends the request in response to receipt of the request, and
the liaison output means selects medical image data on the basis of the patient's chart output from the electronic patient's chart output means by the use of liaison information, sends a request for receiving selected medical image data to the server and causes the image output means to output the medical image data sent from the server upon receipt of the request.

62. A medical information output system as defined in Claim 60 in which
each of the clients is provided with a function of sending a request for receiving medical image data out of image data stored in the image storage means to the server,
the server is provided with a function of sending image data to the client which sends the request in response to receipt of the request, and
the liaison output means selects patient's chart on the basis of the medical image data received from the server by the use of liaison information, and causes the electronic patient's chart output means to output the selected patient's chart.

63. A medical information output system as defined in Claim 60 in which
each of the clients is provided with a function of sending a request for receiving medical image data out of image data stored in the image storage means to the server,
the server is provided with a function of sending image data to the client which sends the request in response to receipt of the request, and
the liaison output means is provided with a function of selecting medical image data on the basis of the patient's chart output from the electronic patient's chart output means by the use of liaison information, sending a request for receiving selected medical image data to the server and causing the image output means to output the medical image data sent from the server upon receipt of the request and a function of selecting patient's chart on the basis of the medical image data received from the server by the use of liaison information and causing the electronic patient's chart output means to output the selected patient's chart.

64. A client for a medical information output system as defined in Claim 60 comprising
an electronic patient's chart storage means which stores data on the patient's chart,
an electronic patient's chart output means which outputs data on the patient's chart stored in the electronic patient's chart storage means,
a data sending means which sends a request for receiving medical image data out of image data stored in a server connected thereto by way of a network,
a data receiving means which receives, from the server, medical image data as requested by the request for receiving medical image data sent by the data sending means,
an image output means which outputs as a visible image the medical image data received by the data receiving means, and
a liaison output means which causes the electronic patient's chart output means and the image output means to output in liaison with each other, wherein
the liaison output means selects medical image data on the basis of the patient's chart output from the electronic patient's chart output means by the use of liaison information, causes the data sending means to send a request for receiving selected medical image data to the server and causes the image output means to output the medical image data sent from the server upon receipt of the request.

65. A client for a medical information output system as defined in Claim 60 comprising
an electronic patient's chart storage means which stores data on the patient's chart,
an electronic patient's chart output means which outputs data on the patient's chart stored in the electronic patient's chart storage means,
a data sending means which sends a request for receiving medical image data out of image data stored in a server connected thereto by way of a network,
a data receiving means which receives, from the server, medical image data as requested by the request for receiving medical image data sent by the data sending means,
an image output means which outputs as a visible image the medical image data received by the data receiving means, and
a liaison output means which causes the electronic patient's chart output means and the image output means to output in liaison with each other, wherein
the liaison output means selects patient's chart on the basis of the medical image data received from the server by the use of liaison information and causes the electronic patient's chart output means to output the selected patient's chart.

66. A client for a medical information output system as defined in Claim 60 comprising
an electronic patient's chart storage means which stores data on the patient's chart, an electronic patient's chart output means which outputs data on the patient's chart stored in the electronic patient's chart storage means, a data sending means which sends a request for receiving medical image data out of image data stored in a server connected thereto by way of a network, a data receiving means which receives, from the server, medical image data as requested by the request for receiving medical image data sent by the data sending means, an image output means which outputs as a visible image the medical image data received by the data receiving means, and a liaison output means which causes the electronic patient's chart output means and the image output means to output in liaison with each other, wherein
the liaison output means is provided with a function of selecting medical image data on the basis of the patient's chart output from the electronic patient's chart output means by the use of liaison information, sending a request for receiving selected medical image data to the server and causing the image output means to output the medical image data sent from the server upon receipt of the request and a function of selecting patient's chart on the basis of the medical image data received from the server by the use of liaison information and causing the electronic patient's chart output means to output the selected patient's chart.

67. A server for a medical information output system as defined in Claim 60 comprising
an image storage means which stores medical image data,
a data receiving means which receives a request for receiving medical image data related to a predetermined examination out of image data stored in the image storage means from a client connected thereto by way of a network, and
a data sending means which sends, to the client, medical image data as requested by the request for receiving medical image data received by the data receiving means.

68. A program for causing a computer to execute
processing of outputting data on the patient's chart stored in a client,
processing of selecting medical image data on the basis of the patient's chart output from the electronic patient's chart output means by the use of liaison information,
processing of sending a request for receiving selected medical image data to a server connected thereto by way of a network,
processing of receiving, from the server, medical image data as requested by the request for receiving medical image data, and
processing outputting the medical image data sent from the server.

69. A program for causing a computer to execute
processing of sending a request for receiving medical image data out of image data stored in a server connected thereto by way of a network,
processing of receiving, from the server, medical image data as requested by the request,
processing of outputting as a visible image the medical image data received,
processing of selecting patient's chart on the basis of the medical image data received from the server by the use of liaison information, and
outputting the selected patient's chart.

70. A program for causing a computer to execute
processing of storing medical image data,
processing of receiving a request for receiving medical image data out of image data stored in the image storage means from a client connected thereto by way of a network, and
processing sending, to the client, medical image data as requested by the request for receiving medical image data received.

71. A medical information output method comprising the steps of
storing data on patient's charts in each of clients equipped in medical facilities,
storing medical image data related to the patient's charts in a server connected to the clients by way of a network, and
causing each of the clients to automatically receive medical image data related to data on a patient's chart to be output and to output the medical image data together with the patient's chart.

72. A medical information output method comprising the steps of
storing data on patient's charts in each of clients equipped in medical facilities,
storing medical image data related to the patient's charts in a server connected to the clients by way of a network, and
causing each of the clients to automatically select data on a patient's chart related to medical image data to be output and to output the selected patient's chart together with the medical image data.
